(19) Europäisches Patentamt
European Patent Office
Office européen des brevets

(11) EP 3 615 186 B1

(12) EUROPEAN PATENT SPECIFICATION

(45) Date of publication and mention of the grant of the patent:
**07.06.2023 Bulletin 2023/23**

(21) Application number: **18791745.5**

(22) Date of filing: **18.04.2018**

(51) International Patent Classification (IPC):
***B01D 57/02*** $^{(2006.01)}$ ***B05D 5/12*** $^{(2006.01)}$
***C07K 1/26*** $^{(2006.01)}$ ***G01N 27/447*** $^{(2006.01)}$
*G01N 30/60* $^{(2006.01)}$ *G01N 30/88* $^{(2006.01)}$

(52) Cooperative Patent Classification (CPC):
**G01N 27/44752; B01D 57/02;** C07K 1/26

(86) International application number:
**PCT/US2018/028091**

(87) International publication number:
**WO 2018/200279 (01.11.2018 Gazette 2018/44)**

# (54) TUNABLE ELECTROOSMOTIC FLOW POLYMER COATED CAPILLARY

POLYMERBESCHICHTETE KAPILLARE MIT ABSTIMMBAREM ELEKTROOSMOTISCHEM DURCHFLUSS

CAPILLAIRE REVÊTU DE POLYMÈRE À FLUX ÉLECTRO-OSMOTIQUE ACCORDABLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.04.2017 US 201762488892 P**

(43) Date of publication of application:
**04.03.2020 Bulletin 2020/10**

(73) Proprietor: **University of Notre Dame du Lac**
**South Bend, Indiana 46617 (US)**

(72) Inventors:
- **DOVICHI, Norman**
  **South Bend, Indiana 46617 (US)**
- **ZHANG, Zhenbin**
  **South Bend, Indiana 46617 (US)**

(74) Representative: **FRKelly**
**27 Clyde Road**
**Dublin D04 F838 (IE)**

(56) References cited:
**WO-A2-01/09204**
**US-A- 5 259 939**
**US-A- 6 074 541**
**US-A1- 2004 045 828**
**US-A1- 2010 087 603**
**US-A1- 2012 000 780**
**US-B1- 7 303 821**

- **ZHENBIN ZHANG ET AL: "Surface-Confined Aqueous Reversible Addition-Fragmentation Chain Transfer (SCARAFT) Polymerization Method for Preparation of Coated Capillary Leads to over 10 000 Peptides Identified from 25 ng HeLa Digest by Using Capillary Zone Electrophoresis-Tandem Mass Spectrometry", ANALYTICAL CHEMISTRY, vol. 89, no. 12, 7 June 2017 (2017-06-07), pages 6774-6780, XP055673774, US ISSN: 0003-2700, DOI: 10.1021/acs.analchem.7b01147**
- **XUEYING HUANG ET AL: "SURFACE-CONFINED LIVING RADICAL POLYMERIZATION FOR COATINGS IN CAPILLARY ELECTROPHORESIS", ANALYTICAL CHEMISTRY, AMERICAN CHEMICAL SOCIETY, US, vol. 70, no. 19, 1 October 1998 (1998-10-01), pages 4023-4029, XP000790311, ISSN: 0003-2700, DOI: 10.1021/AC980231C**
- **JEAN-FRAN?OIS BAUSSARD ET AL: "New chain transfer agents for reversible addition-fragmentation chain transfer (RAFT) polymerisation in aqueous solution", POLYMER, vol. 45, no. 11, 1 May 2004 (2004-05-01), pages 3615-3626, XP055219307, GB ISSN: 0032-3861, DOI: 10.1016/j.polymer.2004.03.081**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- SRINIVASAN K ET AL: "Cross-Linked Polymer Coatings for Capilalry Electrophoresis and Application to Analysis of Basic Proteins, Acidic Proteins, and Inorganic Ions", ANALYTICAL CHEMISTRY, AMERICAN CHEMICAL SOCIETY, US, vol. 69, 15 July 1997 (1997-07-15), pages 2798-2805, XP002568644, ISSN: 0003-2700, DOI: 10.1021/AC970041M
- LIU, Q ET AL.: 'Poly(diallyldimethylammonium chloride) as a Cationic Coating for Capillary . Electrophoresis' JOURNAL OF CHROMATOGRAPHIC SCIENCE vol. 35, no. 3, March 1997, pages 126 - 130, XP055446147

## Description

### RELATED APPLICATIONS

This application claims priority under 35 U.S.C. §1 19(e) to U.S. Provisional Patent Application No. 62/488,892, filed April 24, 2017.

### GOVERNMENT SUPPORT

This invention was made with government support under Grant No. R01GM096767 awarded by the National Institutes of Health. The government has certain rights in the invention.

### BACKGROUND OF THE INVENTION

Capillary zone electrophoresis (CZE) is attracting increased attention for mass spectrometry-based proteomic analysis due to its sensitivity, orthogonality to reversed-phase chromatography, and low cost. While separation is based on the charge-to-size ratio of analyte in CZE, the characteristics of the capillary play important roles in the electrophoretic performance. Non-specific adsorption of analyte to silanol groups on the inner surface of the capillary leads to sample loss, peak tailing, and poor reproducibility. High electroosmotic flow (EOF) produced by the silanol groups results in a relatively short separation window, which limits the number of tandem mass spectra that can be generated during proteomic analysis, which limits the number of peptide identifications.

Various capillary coatings had been developed to overcome these problems. Currently, the most widely used coating method is based on free radical polymerization reactions. In this method, the inner wall of the fused capillary is pretreated by using a bifunctional compound, such as γ-methacryloxypropyltrimethoxysilane, wherein one group reacts specifically with the silanol groups on the inner wall of the capillary and the other with a monomer taking part in a polymerization process. Then, the pretreated capillary is filled with a de-aerated polymerization mixture, typically composed of the acrylamide, N,N,N',N'-tetramethylethylenediamine, and potassium persulphate. This procedure forms a layer of noncrosslinked polyacrylamide, usually referred to as linear polyacrylamide (LPA).

There are several disadvantages to this method. Free radical scavengers can be present at trace levels, leading to irreproducible coating characteristics. In addition, the acrylamide monomer not only reacts with the functional group on the inner wall of the capillary but also reacts with other acrylamide monomers in the solution, and the polymer formed in solution can clog the capillary, leading to the failure of the coating process. Moreover, the film thickness is not predictable.

To overcome these problems, Huang and colleagues (Anal. Chem., 1998, 70, 4023) developed a surface-confined atom transfer radical polymerization (SCATRP) method to covalently bond both linear and cross-linked polymer films to silica. Infrared spectroscopy showed that the film growth is controllable, and atomic force microscopy revealed that smooth films were prepared. CZE of strongly basic proteins using SCATRP-coated capillaries provided the high efficiency expected for polyacrylamide. However, ATRP is governed by a transition-metal-catalyzed activation and deactivation equilibrium, and traditional ATRP systems require high catalyst concentration to maintain activity throughout the polymerization. It is difficult to remove the transition-metal ions after the polymerization reaction; the presence of residual amounts of the metal catalyst in polymers often raises concerns in biomedical applications, and bipyridine adsorption during ATRP would resulted in anodic EOF during CZE at low pH.

In contrast, reversible addition-fragmentation chain transfer (RAFT) polymerization avoids use of metal catalysts and is tolerant of a wide variety of reaction conditions and functionalities. Ali and Cheong (J. Sep. Sci., 2015, 38, 1763) reported a method for the immobilization of N-phenylacrylamide-styrene copolymer on the inner surface of capillaries with RAFT polymerization for applications in capillary electrochromatograpy (CEC). A ligand with a terminal halogen (4-chloromethylphenyl isocyanate), or 4-(trifluoromethoxy) phenyl isocyanate was bound to the inner surface of a pretreated silica capillary in the presence of dibutyltin dichloride as a catalyst through an isocyanate-hydroxyl reaction. Attachment of initiator (sodium diethyl dithiocarbamate) to the bound ligand was carried out and followed by *in situ* polymerization. The resulting capillary showed good separation performance for derivatized saccharide isomers and tryptic digest of cytochrome C in CEC with UV detection. The structure of the prepared block copolymer is not clear because all the monomers were added to the polymerization mixture before the RAFT polymerization reaction. In addition, the living characteristic of the resulted polymer was not demonstrated. Moreover, the reaction was carried out in organic solvents (toluene or p-xylene), which are not environment-friendly. Finally, the RAFT-polymerized coated column was not evaluated for proteomics applications in CZE.

WO0109204 concerns controlled architecture polymers made preferably with acrylamide type monomers are prepared in living-type or semi-living-type free radical polymerizations, with the architecture preferably being other than linear, such as star, branched, grafted or hyper-branched. The controlled architecture polymers have high weight average

molecular weights and low viscosities, which make them particularly useful in replaceable capillary electrophoresis separation media for biological molecules, such as DNA fragments.

US6074541 concerns a method of coating a solid support (e.g. a capillary or chromatography packing) to alter the properties of the support surface for separating components in a fluid stream. The method comprises (a) covalently binding a coupling agent (including functional groups capable of forming free radical sites under hydrogen abstraction conditions) to the support surface in a uniform layer, and (b) thereafter, contacting the bound coupling agent with a solution of preformed polymer comprising totally saturated carbon chain backbones including leaving groups, under hydrogen abstraction conditions of elevated temperature in the presence of a free radical catalyst to remove leaving groups from the carbon chains to form free radical carbon binding sites which covalently bond to the coupling agent layer and to crosslink at least some of the preformed polymer through the free radical carbon binding sites to form a dimensional polymer network coating on said solid support surface. Alternatively, the coating is applied directly to an organic solid support without an intermediate coupling agent.

Zhenbin Zhang et al. concerns a surface-confined aqueous reversible addition-fragmentation chain transfer (SCARAFT) polymerization method for preparation of coated capillary leads to over 10 000 peptides identified from 25 ng HeLa digest by using capillary zone electrophoresis-tandem mass spectrometry (Analytical Chemistry, US, vol. 89, no. 12, pages 6774 - 6780).

Xueying Huang et al. concerns surface-confined living radical polymerization for coatings in capillary electrophoresis (Analytical Chemistry, American Chemical Society, US, vol. 70, no. 19, pages 4023 - 4029).

Jean-Francois Baussard et al. concerns new chain transfer agents for reversible addition-fragmentation chain transfer (RAFT) polymerisation in aqueous solution (Polymer, GB, vol. 45, no. 11, pages 3615 - 3626).

Srinivasan K et al. concerns cross-linked polymer coatings for capillary electrophoresis and application to analysis of basic proteins, acidic proteins, and inorganic ions (Analytical Chemistry, American Chemical Society, US, vol. 69, pages 2798 - 2805).

The problem is the performance of polymer coated capillaries for the separation of proteins or protein digests by CZE needs to be improved. Accordingly, there is a need for coatings with low EOF that meet the demands of resolving substances in a proteomic analysis, as well as coatings with charged (high EOF) that minimize the nonspecific adsorption of extremely basic and acidic analytes and improve the throughput.

## SUMMARY

In this paper, we describe an environmentally friendly coating method based on surface-confined aqueous reversible addition-fragmentation chain transfer (SCARAFT) polymerization reaction for covalently bonding polymers to capillary inner surfaces. To illustrate that the SCARAFT method is applicable to various vinyl monomers, both neutral and positively charged coatings were prepared. Moreover, the living characteristic of the coating prepared by the SCARAFT method was confirmed by preparing a block copolymer coating, wherein a neutral coating and a positively charged coating were prepared sequentially. The coated capillaries were evaluated by measuring the EOF and by analyzing tryptic digests and a protein mixture with CZE.

Accordingly, a first aspect of the present invention provides a polymer coated separation capillary comprising:

a) a fused silica capillary;
b) a coating comprising a chain transfer moiety covalently bonded to the inner surface of the fused silica capillary; and
c) a substituted polyethylene polymer covalently bonded to the chain transfer moiety wherein the polyethylene units of the polymer are substituted by -C(=O)G,

wherein G is $NR_2$, or OR; R is H, $(C_1-C_6)$alkyl, or $(C_1-C_6)$alkyl-$N(R^a)_3X$; each $R^a$ is independently H, $(C_1-C_6)$alkyl, or aryl; and is X is a counter ion;
wherein the polymer coated separation capillary has an electroosmotic flow of about $0.1 \times 10^{-6}$ $cm^2$ $V^{-1}$ $s^{-1}$ to about $10 \times 10^{-4}$ $cm^2$ $V^{-1}$ $s^{-1}$ for capillary zone electrophoresis, and the coating is uncontaminated by metals and free radical scavengers.

This disclosure provides a coated separation capillary wherein the coating comprises a polymer of Formula I or Formula II:

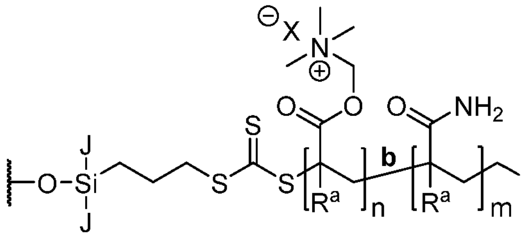

(I),

or

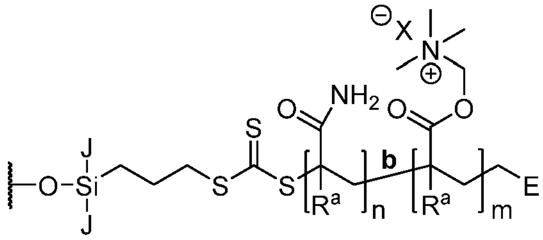

(II);

wherein

J is methoxy, ethoxy or halo;
X is halo;
E is H, aryl, alkyl, cyano;
n is 0 to 10,000; and
m is 0 to 10,000, wherein n and m cannot both be 0.

Additionally, a second aspect of the present invention provides a method of fabricating the polymer coated separation capillary of claim 1 comprising:

a) contacting the inner surface of the fused silica capillary with a chain transfer reagent to provide a covalently modified inner surface of the fused silica capillary;
b) contacting the modified inner surface with an aqueous mixture of a radical initiator, a substituted vinyl monomer, and an optional second monomer; and
c) initiating a living radical polymerization by heating or irradiating the mixture; thereby fabricating the polymer coated separation capillary.

Polymer coatings of Formulas I and IA and Formulas II and IIA, intermediates for the synthesis of polymer coatings of Formulas I and IA and Formulas II and IIA, as well as methods of preparing polymer coatings of Formulas I and IA and Formulas II and IIA are disclosed. Polymer coatings of Formulas I and IA and Formulas II and IIA that are useful as intermediates for the synthesis of other useful polymer coatings are also disclosed.

## BRIEF DESCRIPTION OF THE DRAWINGS

The following drawings form part of the specification and are included to further demonstrate certain embodiments or various aspects of the invention. In some instances, embodiments of the invention can be best understood by referring to the accompanying drawings in combination with the detailed description presented herein. The description and accompanying drawings may highlight a certain specific example, or a certain aspect of the invention. However, one skilled in the art will understand that portions of the example or aspect may be used in combination with other examples or aspects of the invention, insofar as they are within the scope of the appended claims.

**Figure 1.** Schematic diagram for preparation of the coated capillary by SCARAFT.
**Figure 2.** Peptides (A) and protein group (B) overlaps between the CZE and UPLC analysis of *E. coli* tryptic digests.
**Figure 3.** Representative electrophorogram of CZE-ESI-MS/MS analysis of the HeLa digest. Experimental conditions: Q Exactive HF mass spectrometer; 50 $\mu$m i.d. $\times$ 150 $\mu$m o.d. $\times$ 100 cm long LPA coated capillary; 25 ng HeLa digest; 1 M HAc separation buffer; 21.6 kV separation voltage; and 1.6 kV spray voltage.
**Figure 4.** pI distribution of the identified peptides from HeLa digest. Experimental conditions are same as **Figure 3.**

**Figure 5.** Separation of intact proteins by using a capillary with the direct positively charged coating (A), and block copolymer coating (B). Peak: (1) carbonic anhydrase, (2) myoglobin, (3) ribonuclease A, (4) lysozyme. Experimental conditions: 50 μm i.d. × 150 μm o.d. × 62 cm long; 1 M HAc background electrolyte; 1.6 kV spray voltage, -19.6 kV separation voltage.

**Figure 6.** A representative chromatogram for UPLC-ESI-MS/MS analysis of the *E. coli* digest. Experimental conditions: LTQ Orbitrap Velos mass spectrometer, a commercial C18 reversed phase column (Waters, 100 μm ×100 mm, 1.7 μm particle, BEH130C18), 50 ng *E.coli* digest, Buffer A (0.1% FA in water) and buffer B (0.1% FA in ACN) were used as mobile phases for gradient separation: 0-10 min, 2% B; 10-11 min, 2-8% B; 11-71 min, 8-30% B; 71-72 min, 30-80% B; 72-77 min, 80% B; 77-78 min, 80-2% B; 78-90 min, 2% B.

**Figure 7.** Detector trace for μEOF determination. (A) First coated capillary. (B) Second coated capillary. (C) Third coated capillary. Experiment conditions: BGE, 1 M HAc, capillary: 50 μm i.d.×150 μm o.d., L= 100 cm, tinj= 2 s, ttr=40 s, tmigr = 50 min. Peaks are benzyl alcohol. 1.6 kV spray voltage. 26.6 kV separation voltage.

**Figure 8.** Representative electropherograms for the testing of the long-term stability and carryover of the LPA coated capillary. Experimental conditions: LTQ XL mass spectrometer; 50 μm i.d. × 150 μm o.d. × 100 cm long LPA coated capillary; 25 nL of 2 mg/mL *Xenopus laevis* digest in 30 mM NH4HCO3; for blank analysis, 25 nL of 1 M HAc was injected; 1 M HAc separation buffer; 26.6 kV separation voltage; and 1.6 kV spray voltage.

**Figure 9.** Cumulative distribution of the migration time of the peptides identified from HeLa digest. Experimental conditions: Q Exactive HF mass spectrometer; 50 μm i.d. × 150 μm o.d. × 100 cm long LPA coated capillary; 25 ng HeLa digest; 1 M HAc separation buffer; 21.6 kV separation voltage; and 1.6 kV spray voltage.

**Figure 10.** The migration time of the peptides with various pI. Experimental conditions are same as **Figure 8.**

**Figure 11.** Migration time distribution of the identified phosphopeptides. Experimental conditions are same as **Figure 8.**

**Figure 12.** The pI distribution of the phosphopeptides. Experimental conditions are same as **Figure 8.**

## DETAILED DESCRIPTION

A surface-confined aqueous reversible addition-fragmentation chain transfer (SCARAFT) polymerization method was developed to coat capillaries for use in capillary zone electrophoresis (CZE). SCARAFT polymerization primarily takes place on the inner surface of the capillary instead of in solution, which greatly improves the homogeneity of the coating. Capillaries treated with this coating produced an electroosmotic mobility of $2.8 \pm 0.2 \times 10^{-6}$ $cm^2 \cdot V^{-1} \cdot s^{-1}$ (N = 3), which is roughly an order of magnitude lower than that of commercial linear polyacrylamide (LPA)-coated capillaries. Coated capillaries were evaluated for bottom-up proteomic analysis using CZE. The very low electroosmotic mobility results in a 200 min separation and improved single-shot analysis. An average of 977 protein groups and 5605 unique peptides were identified from 50 ng of an E. coli digest, and 2158 protein groups and 10 005 peptides were identified from 25 ng of a HeLa digest using single-shot analysis with a SCARAFT-acrylamide capillary coupled to a Q Exactive HF mass spectrometer. The coating is stable. A single capillary was used for over 200 h (8.4 days) of continuous operation. RSD in migration time was between 2 and 3% for selected ion electropherograms (SIEs) generated for six ions; median theoretical plate counts ranged from 240 000 to 600 000 for these SIEs. Various types of coatings were prepared by simply changing the functional vinyl monomers in the polymerization mixture. Positively charged coatings using direct attachment and formation of a block copolymer were prepared and demonstrated for the separation of mixtures of intact proteins.

Definitions

The following definitions are included to provide a clear and consistent understanding of the specification and claims. As used herein, the recited terms have the following meanings. All other terms and phrases used in this specification have their ordinary meanings as one of skill in the art would understand. Such ordinary meanings may be obtained by reference to technical dictionaries, such as Hawley's Condensed Chemical Dictionary 14th Edition, by R.J. Lewis, John Wiley & Sons, New York, N.Y., 2001.

References in the specification to "one embodiment", "an embodiment", etc., indicate that the embodiment described may include a particular aspect, feature, structure, moiety, or characteristic, but not every embodiment necessarily includes that aspect, feature, structure, moiety, or characteristic. Moreover, such phrases may, but do not necessarily, refer to the same embodiment referred to in other portions of the specification. Further, when a particular aspect, feature, structure, moiety, or characteristic is described in connection with an embodiment, it is within the knowledge of one skilled in the art to affect or connect such aspect, feature, structure, moiety, or characteristic with other embodiments, whether or not explicitly described.

The singular forms "a," "an," and "the" include plural reference unless the context clearly dictates otherwise. Thus, for example, a reference to "a compound" includes a plurality of such compounds, so that a compound X includes

a plurality of compounds X. It is further noted that the claims may be drafted to exclude any optional element. As such, this statement is intended to serve as antecedent basis for the use of exclusive terminology, such as "solely," "only," and the like, in connection with any element described herein, and/or the recitation of claim elements or use of "negative" limitations.

The term "and/or" means any one of the items, any combination of the items, or all of the items with which this term is associated. The phrases "one or more" and "at least one" are readily understood by one of skill in the art, particularly when read in context of its usage. For example, the phrase can mean one, two, three, four, five, six, ten, 100, or any upper limit approximately 10, 100, or 1000 times higher than a recited lower limit. For example, one or more substituents on a phenyl ring refers to one to five, or one to four, for example if the phenyl ring is substituted.

As will be understood by the skilled artisan, all numbers, including those expressing quantities of ingredients, properties such as molecular weight, reaction conditions, and so forth, are approximations and are understood as being optionally modified in all instances by the term "about." These values can vary depending upon the desired properties sought to be obtained by those skilled in the art utilizing the teachings of the descriptions herein. It is also understood that such values inherently contain variability necessarily resulting from the standard deviations found in their respective testing measurements. When values are expressed as approximations, by use of the antecedent "about," it will be understood that the particular value without the modifier "about" also forms a further aspect.

The terms "about" and "approximately" are used interchangeably. Both terms can refer to a variation of ± 5%, ± 10%, ± 20%, or ± 25% of the value specified. For example, "about 50" percent can in some embodiments carry a variation from 45 to 55 percent, or as otherwise defined by a particular claim. For integer ranges, the term "about" can include one or two integers greater than and/or less than a recited integer at each end of the range. Unless indicated otherwise herein, the terms "about" and "approximately" are intended to include values, e.g., weight percentages, proximate to the recited range that are equivalent in terms of the functionality of the individual ingredient, composition, or embodiment. The terms "about" and "approximately" can also modify the end-points of a recited range as discussed above in this paragraph.

As will be understood by one skilled in the art, for any and all purposes, particularly in terms of providing a written description, all ranges recited herein also encompass any and all possible sub-ranges and combinations of sub-ranges thereof, as well as the individual values making up the range, particularly integer values. It is therefore understood that each unit between two particular units are also disclosed. For example, if 10 to 15 is disclosed, then 11, 12, 13, and 14 are also disclosed, individually, and as part of a range. A recited range (e.g., weight percentages or carbon groups) includes each specific value, integer, decimal, or identity within the range. Any listed range can be easily recognized as sufficiently describing and enabling the same range being broken down into at least equal halves, thirds, quarters, fifths, or tenths. As a non-limiting example, each range discussed herein can be readily broken down into a lower third, middle third and upper third, etc. As will also be understood by one skilled in the art, all language such as "up to", "at least", "greater than", "less than", "more than", "or more", and the like, include the number recited and such terms refer to ranges that can be subsequently broken down into sub-ranges as discussed above. In the same manner, all ratios recited herein also include all sub-ratios falling within the broader ratio. Accordingly, specific values recited for radicals, substituents, and ranges, are for illustration only; they do not exclude other defined values or other values within defined ranges for radicals and substituents. It will be further understood that the endpoints of each of the ranges are significant both in relation to the other endpoint, and independently of the other endpoint.

One skilled in the art will also readily recognize that where members are grouped together in a common manner, such as in a Markush group, the invention encompasses not only the entire group listed as a whole, but each member of the group individually and all possible subgroups of the main group. Additionally, for all purposes, the invention encompasses not only the main group, but also the main group absent one or more of the group members. The invention therefore envisages the explicit exclusion of any one or more of members of a recited group. Accordingly, provisos may apply to any of the disclosed categories or embodiments whereby any one or more of the recited elements, species, or embodiments, may be excluded from such categories or embodiments, for example, for use in an explicit negative limitation.

The term "contacting" refers to the act of touching, making contact, or of bringing to immediate or close proximity. For example, to bring about a chemical reaction, or a physical change, e.g., in a solution, in a reaction mixture.

The term "substantially" as used herein, is a broad term and is used in its ordinary sense, including, without limitation, being largely but not necessarily wholly that which is specified. For example, the term could refer to a numerical value that may not be 100% the full numerical value. The full numerical value may be less by about1%, about 2%, about 3%, about 4%, about 5%, about 6%, about 7%, about 8%, about 9%, about 10%, about 15%, or about 20%.

As used herein, the term "substituted" or "substituent" is intended to indicate that one or more (for example., 1-20 in various embodiments, 1-10 in other embodiments, 1, 2, 3, 4, or 5; in some embodiments 1, 2, or 3; and in other embodiments 1 or 2) hydrogens on the group indicated in the expression using "substituted" (or "substituent") is replaced with a selection from the indicated group(s), or with a suitable group known to those of skill in the art, provided that the indicated atom's normal valency is not exceeded, and that the substitution results in a stable compound. Suitable indicated

groups include, e.g., alkyl, alkenyl, alkynyl, alkoxy, halo, haloalkyl, hydroxy, hydroxyalkyl, aryl, heteroaryl, heterocycle, cycloalkyl, alkanoyl, alkoxycarbonyl, amino, alkylamino, dialkylamino, trifluoromethylthio, difluoromethyl, acylamino, nitro, trifluoromethyl, trifluoromethoxy, carboxy, carboxyalkyl, keto, thioxo, alkylthio, alkylsulfinyl, alkylsulfonyl, and cyano. Additionally, non-limiting examples of substituents that can be bonded to a substituted carbon (or other) atom include F, Cl, Br, I, OR', OC(O)N(R')$_2$, CN, $CF_3$, $OCF_3$, R', O, S, C(O), S(O), methylenedioxy, ethylenedioxy, N(R')$_2$, SR', SOR', $SO_2$R', $SO_2$N(R')$_2$, $SO_3$R', C(O)R', C(O)C(O)R', C(O)$CH_2$C(O)R', C(S)R', C(O)OR', OC(O)R', C(O)N(R')$_2$, OC(O)N(R')$_2$, C(S)N(R')$_2$, $(CH_2)_{0-2}$NHC(O)R', N(R')N(R')C(O)R', N(R')N(R')C(O)OR', N(R')N(R')CON(R')$_2$, N(R')$SO_2$R', N(R')$SO_2$N(R')$_2$, N(R')C(O)OR', N(R')C(O)R', N(R')C(S)R', N(R')C(O)N(R')$_2$, N(R')C(S)N(R')$_2$, N(COR')COR', N(OR')R', C(=NH)N(R')$_2$, C(O)N(OR')R', or C(=NOR')R' wherein R' can be hydrogen or a carbon-based moiety, and wherein the carbon-based moiety can itself be further substituted. When a substituent is monovalent, such as, for example, F or Cl, it is bonded to the atom it is substituting by a single bond. When a substituent is more than monovalent, such as O, which is divalent, it can be bonded to the atom it is substituting by more than one bond, i.e., a divalent substituent is bonded by a double bond; for example, a C substituted with O forms a carbonyl group, C=O, wherein the C and the O are double bonded. Alternatively, a divalent substituent such as O, S, C(O), S(O), or S(O)$_2$ can be connected by two single bonds to two different carbon atoms. For example, O, a divalent substituent, can be bonded to each of two adjacent carbon atoms to provide an epoxide group, or the O can form a bridging ether group between adjacent or non-adjacent carbon atoms, for example bridging the 1,4-carbons of a cyclohexyl group to form a [2.2.1]-oxabicyclo system. Further, any substituent can be bonded to a carbon or other atom by a linker, such as $(CH_2)_n$ or $(CR'_2)_n$ wherein n is 1, 2, 3, or more, and each R' is independently selected.

The term "alkyl" refers to a branched or unbranched hydrocarbon having, for example, from 1-20 carbon atoms, and often 1-12, 1-10, 1-8, 1-6, or 1-4 carbon atoms. As used herein, the term "alkyl" also encompasses a "cycloalkyl", defined below. Examples include, but are not limited to, methyl, ethyl, 1-propyl, 2-propyl (iso-propyl), 1-butyl, 2-methyl-1-propyl (*isobutyl*), 2-butyl (see-butyl), 2-methyl-2-propyl (t-butyl), 1-pentyl, 2-pentyl, 3-pentyl, 2-methyl-2-butyl, 3-methyl-2-butyl, 3-methyl-1-butyl, 2-methyl-1-butyl, 1-hexyl, 2-hexyl, 3-hexyl, 2-methyl-2-pentyl, 3-methyl-2-pentyl, 4-methyl-2-pentyl, 3-methyl-3-pentyl, 2-methyl-3-pentyl, 2,3-dimethyl-2-butyl, 3,3-dimethyl-2-butyl, hexyl, octyl, decyl, dodecyl, and the like. The alkyl can be unsubstituted or substituted, for example, with a substituent described below. The alkyl can also be optionally partially or fully unsaturated. As such, the recitation of an alkyl group can include both alkenyl and alkynyl groups. The alkyl can be a monovalent hydrocarbon radical, as described and exemplified above, or it can be a divalent hydrocarbon radical (i.e., an alkylene).

The term "aryl" refers to an aromatic hydrocarbon group derived from the removal of at least one hydrogen atom from a single carbon atom of a parent aromatic ring system. The radical attachment site can be at a saturated or unsaturated carbon atom of the parent ring system. The aryl group can have from 6 to 30 carbon atoms, for example, about 6-10 carbon atoms. In other embodiments, the aryl group can have 6 to 60 carbons atoms, 6 to 120 carbon atoms, or 6 to 240 carbon atoms. The aryl group can have a single ring (e.g., phenyl) or multiple condensed (fused) rings, wherein at least one ring is aromatic (e.g., naphthyl, dihydrophenanthrenyl, fluorenyl, or anthryl). Typical aryl groups include, but are not limited to, radicals derived from benzene, naphthalene, anthracene, biphenyl, and the like. The aryl can be unsubstituted or optionally substituted.

Substituents of the compounds and polymers described herein may be present to a recursive degree. In this context, "recursive substituent" means that a substituent may recite another instance of itself. Because of the recursive nature of such substituents, theoretically, a large number may be present in any given claim. One of ordinary skill in the art of organic chemistry understands that the total number of such substituents is reasonably limited by the desired properties of the compound intended. Such properties include, by of example and not limitation, physical properties such as molecular weight, solubility or log P, application properties such as activity against the intended target, and practical properties such as ease of synthesis. Recursive substituents are an intended aspect of the invention. One of ordinary skill in the art of organic chemistry understands the versatility of such substituents. To the degree that recursive substituents are present in a claim of the invention, the total number in the repeating unit of a polymer example can be, for example, about 1-50, about 1-40, about 1-30, about 1-20, about 1-10, or about 1-5.

The term, "repeat unit", "repeating unit", or "block" as used herein refers to the moiety of a polymer that is repetitive. The repeat unit may comprise one or more repeat units, labeled as, for example, repeat unit A, repeat unit B, repeat unit C, etc. Repeat units A-C, for example, may be covalently bound together to form a combined repeat unit. Monomers or a combination of one or more different monomers can be combined to form a (combined) repeat unit of a polymer or copolymer.

The term "molecular weight" for the copolymers disclosed herein refers to the average number molecular weight (Mn). The corresponding weight average molecular weight (Mw) can be determined from other disclosed parameters by methods (e.g., by calculation) known to the skilled artisan.

The copolymers disclosed herein can comprise random "r" or block "b" copolymers. The ends of the polymer or copolymer (i.e., the initiator end or terminal end),may bes a low molecular weight moiety (e.g. under 500 Da), such as, H, OH, OOH, $CH_2OH$, CN, $NH_2$, or a hydrocarbon such as an alkyl (for example, a butyl or 2-cyanoprop-2-yl moiety

at the initiator and terminal end), alkene or alkyne, or a moiety as a result of an elimination reaction at the first and/or last repeat unit in the copolymer, unless stated otherwise.

Embodiments of the Invention

In a first aspect, the present invention provides various embodiments of a polymer coated separation capillary comprising:

a) a fused silica capillary;
b) a coating comprising a chain transfer moiety covalently bonded to the inner surface of the fused silica capillary; and
c) a substituted polyethylene polymer covalently bonded to the chain transfer moiety wherein the polyethylene units of the polymer are substituted by -C(=O)G,

wherein G is $NR_2$, or OR; R is H, $(C_1-C_6)$alkyl, or $(C_1-C_6)$alkyl-$N(R^a)_3X$; each $R^a$ is independently H, $(C_1-C_6)$alkyl, or aryl; and is X is a counter ion;
wherein, optionally, the polymer coated separation capillary has an electroosmotic flow of about $0.1 \times 10^{-6}$ $cm^2$ $V^{-1}$ $s^{-1}$ to about $10 \times 10^{-4}$ $cm^2$ $V^{-1}$ $s^{-1}$ for capillary zone electrophoresis, and the coating is uncontaminated by metals and free radical scavengers.

In additional embodiments, the separation capillary of claim 1 wherein the electroosmotic flow (EOF) is about $1 \times 10^{-6}$ $cm^2$ $V^{-1}$ $s^{-1}$ to about $10 \times 10^{-6}$ $cm^2$ $V^{-1}$ $s^{-1}$. In other embodiments, the EOF is $0.1 \times 10^{-7}$ $cm^2$ $V^{-1}$ $s^{-1}$ to about $10 \times 10^{-7}$ $cm^2$ $V^{-1}$ $s^{-1}$, $0.1 \times 10^{-6}$ $cm^2$ $V^{-1}$ $s^{-1}$ to about $10 \times 10^{-6}$ $cm^2$ $V^{-1}$ $s^{-1}$, $0.1 \times 10^{-5}$ $cm^2$ $V^{-1}$ $s^{-1}$ to about $10 \times 10^{-5}$ $cm^2$ $V^{-1}$ $s^{-1}$, $0.1 \times 10^{-4}$ $cm^2$ $V^{-1}$ $s^{-1}$ to about $10 \times 10^{-4}$ $cm^2$ $V^{-1}$ $s^{-1}$.

In other embodiments, -C(=O)G is -C(=O)$NH_2$. In other embodiments, -C(=O)G is -C(=O)$OCH_2N(CH_3)_3X$. In yet other embodiments, the polymer comprises a random polymer or block copolymer of -C(=O)$NH_2$ substituted ethylene units and --C(=O)$OCH_2N(CH_3)_3$ substituted ethylene units. In additional embodiments, the separation capillary comprises about 200,000 to about 800,000 theoretical plates. In other embodiments the number of theoretical plates is about 200,000 to about 300,000, about 300,000 to about 400,000, about 400,000 to about 500,000, about 500,000 to about 600,000, about 700,000 to about 800,000, about 800,000 to about 1.000,000.

In various embodiments, the chain transfer moiety comprises -$SiJ_2(CH_2)_3$SC(=S)S-, wherein each J is independently H, G, or halo, or other chain transfer agents, such as but not limited to chain transfer agents comprising --SC(=S)S-. In additional embodiments, the separation capillary comprises about 200,000 to about 800,000 theoretical plates, the electroosmotic flow is about $1 \times 10^{-6}$ $cm^2$ $V^{-1}$ $s^{-1}$ to about $10 \times 10^{-6}$ $cm^2$ $V^{-1}$ $s^{-1}$, and -C(=O)G is -C(=O)$NR_2$. In yet other embodiments, R is H.

The disclosure provides various embodiments of a polymer coated separation capillary wherein the coating comprises a polymer of Formula I or Formula II:

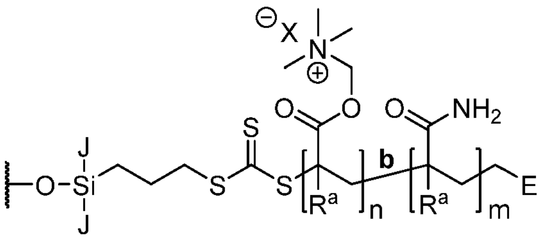

(I),

or

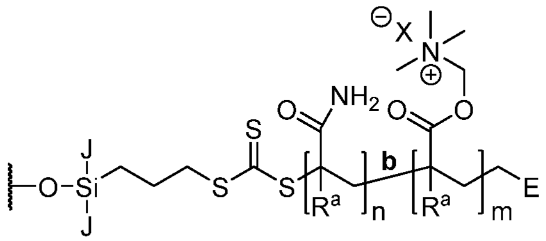

(II);

wherein

J is methoxy, ethoxy or halo;
X is halo;
E is H, aryl, alkyl, cyano;
n is 0 to 10,000; and
m is 0 to 10,000, wherein n and m cannot both be 0.

In various embodiments, the symbol b of Formula I or Formula II indicates that Formula I or Formula II is a block copolymer when n and m are each greater than 1. In other embodiments, n is 0 to 1000, 1 to 500, or 500 to 2000. In yet other embodiments, m is 0 to 1000, 1 to 500, or 500 to 2000.

In a second aspect, the present invention provides additionally provides a method of fabricating the above described polymer coated separation capillary comprising:

a) contacting the inner surface of the fused silica capillary with a chain transfer reagent to provide a covalently modified inner surface of the fused silica capillary;
b) contacting the modified inner surface with an aqueous mixture of a radical initiator, a substituted vinyl monomer, and an optional second monomer; and
c) initiating a living radical polymerization by heating or irradiating the mixture; thereby fabricating the polymer coated separation capillary.

In additional embodiments, the aqueous mixture comprises a buffer. In other embodiments, the concentration of the radical initiator is about $10^{-5}$ molar to about $10^{-4}$ molar. In yet other embodiments, concentration of the radical initiator is about $10^{-6}$ molar to about $10^{-5}$ molar, about $10^{-5}$ molar to about $10^{-3}$ molar, or about $10^{-4}$ molar to about $10^{-3}$ molar. In other embodiments, the concentration of the substituted vinyl monomer is about 0.1 molar to about 2 molar.

In various embodiments, the polymer coated separation capillary has a neutral charge. In additional embodiments, the polymer coated separation capillary is positively charged. In yet other embodiments, the polymer coated separation capillary is negatively charged.

In some embodiments the separation capillary comprises the substituted vinyl monomer and the second monomer. In other embodiments the second monomer is also a substituted vinyl monomer. In other embodiments, the radical initiator comprises a halogen molecule, an azo compound, an organic peroxide, or an inorganic peroxide.

In various additional embodiments, the polymer coated separation capillary is coated on its inner surface with a block copolymer. In other embodiments, the substituted vinyl monomer is substituted by -C(=O)G, and the optional second monomer is substituted by - C(=O)G. In additional embodiments the substituted vinyl monomer or the optional second monomer is substituted with the substituents described above and may have more than one substituent. In other various embodiments of the described methods above, a living radical polymerization in the polymer coated separation capillary is reinitiated by repeating steps b) and c) with the second monomer. In some other embodiments, initiating the living radical polymerization by heating the mixture is performed at a heating temperature just above room temperature. In other embodiments, the heating temperature is about 0 °C to about 400 °C, about 20 °C to about 80 °C, about 50 °C to about 100 °C, about 50 °C to about 150 °C, about 80 °C to about 200 °C, or about 120 °C to about 300 °C. In some other embodiments, initiating the living radical polymerization by irradiating the mixture is performed at wavelengths in the visible region of the electromagnetic spectrum.

The disclosure additionally provides various embodiments of a polymer coated separation capillary comprising an inner surface coated with a polymer of Formula IA or Formula IIA:

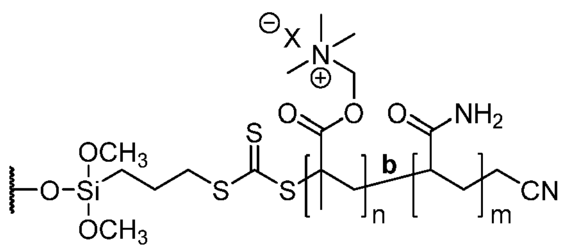
(IA),

or wherein

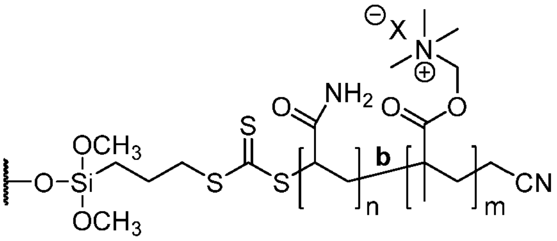

(IIA);

X is halo;
n is 0 to 10,000; and
m is 0 to 10,000 wherein n and m cannot both be 0.

In various embodiments, the symbol b of Formula IA or Formula IIA indicates that Formula IA or Formula IIA is a block copolymer when n and m are each greater than 1.

In other various embodiments, the polymer coated separation capillary performs reproducible separations by capillary zone electrophoresis (CZE) of at least 5,000 identifiable peptides for at least 100 hours of continuous operation. In other embodiments, the polymer coated separation capillary performs reproducible separations by CZE of about 5,000 to about 50,000, about 7,500 to about 15,000, about 10,000 to about 20,000, or about 15,000 to about 30,000 identifiable peptides. In other embodiments, the polymer coated separation capillary performs reproducibly or is stable for about 100 hours to about 100,000 hours of continuous operation, about 200 hours to about 500.hours, or about 1000 hours to about 10,000 hours of continuous operation.

This disclosure provides ranges, limits, and deviations to variables such as volume, mass, percentages, ratios, etc. It is understood by an ordinary person skilled in the art that a range, such as "number1" to "number2", implies a continuous range of numbers that includes the whole numbers and fractional numbers. For example, 1 to 10 means 1, 2, 3, 4, 5, ... 9, 10. It also means 1.0, 1.1, 1.2. 1.3, ..., 9.8, 9.9, 10.0, and also means 1.01, 1.02, 1.03, and so on. If the variable disclosed is a number less than "number10", it implies a continuous range that includes whole numbers and fractional numbers less than number10, as discussed above. Similarly, if the variable disclosed is a number greater than "number10", it implies a continuous range that includes whole numbers and fractional numbers greater than number10. These ranges can be modified by the term "about", whose meaning has been described above.

Results and Discussion

**Preparation of the coated capillaries by surface-confined aqueous reversible-addition fragmentation transfer (SCARFT) polymerization method**

The schematic diagram for preparation of the LPA coated capillary by SCARAFT method is shown in **Figure 1.** The fused silica capillary (50 μm i.d.×150 μm o.d.) was pretreated by washing with 0.1 M NaOH for 2 h, flushing with water until the outflow reached pH -7.0, flushing with 0.1 M HCl for 12 h, and flushing again with water until the outflow reached pH -7.0. The capillary was finally dried with a nitrogen stream overnight at room temperature. Then, the capillary was filled with 50% cyanomethyl [3-(trimethoxysilyl)propyl] trithiocarbonate solution in MeOH(v/v) and incubated in a water bath at 45 °C for 12 h. The capillary was then rinsed with MeOH to flush out the residual reagent and dried with a nitrogen stream. At this point, the inner wall of the capillary was modified by a layer of cyanomethyl [3-(trimethoxysilyl)propyl] trithiocarbonate, which is the chain transfer agent for subsequent attachment of polymer to the wall during the SCARAFT polymerization reaction. For coating the capillary, a modified method from a report was used (Macromol. Rapid Commun. 2011, 32, 958). A prepolymerizable mixture was prepared by mixing acrylamide (0.56 M) and 4,4-azobis(4-cyanovaleric acid) ($5.42 \times 10^{-4}$ M) in an acetate buffer (pH 5.2, 0.27 M acetic acid, and 0.73 M sodium acetate) at room temperature and stirring for about 10 min under nitrogen to form a homogeneous solution. The mixture then was introduced into the pretreated capillary and incubated at and 60 °C for various reaction times. The coated capillary was flushed with MeOH to remove residuals in the capillary and conditioned with the background electrolyte before CZE analysis.

**Effect of Concentration**

The monomer concentration, initiator concentration, the reaction time affect the coating process. Generally, increasing the concentration of the monomer and the initiator increase of the coating thickness and decrease in the required reaction time. For optimization experiments, we kept the initiator concentration at $2.17\times10^{-5}$ M and the reaction

time at 21 h, and prepared the coated capillary with different monomer concentrations. The EOF decreased from $2.4\times10^{-5}$ $cm^2$-$V^{-1}$ -$s^{-1}$ to $8.62 \times 10^{-6}$ $cm^2 \cdot V^{-1} \cdot s^{-1}$ (N=2, RSD=0.9%) when the monomer concentration increased from 0.39 M to 0.56 M. 390 protein groups and 1,923 peptides were identified from 50 ng *E. coli* digest using a coated capillary prepared with 0.39 M monomer. In contrast, 525 protein groups and 2,742 peptides were identified from 50 ng *E. coli* digest using a coated capillary prepared with 0.56 M monomer. The identified protein groups and peptides are listed in the Examples. Increasing the monomer concentration leads to increased free radical polymerization between the monomers, which can result in a clogged capillary. Therefore, the monomer concentration of 0.56 M was used in subsequent studies.

The effect of the initiator concentration on the coating was also studied. Capillaries were prepared with various initiator concentrations while keeping the monomer concentration (0.56 M) unchanged. When the initiator concentration was less than $2.17\times10^{-5}$ M, over 21 hours were required to complete the coating. Initiator concentrations much higher than $2.17\times10^{-3}$ M led to the free radical polymerization reaction between the monomers in the solution, and the capillary was clogged after 21 h. Based on the experiment results, the initiator concentration of $5.42\times10^{-4}$ M was used in our work.

We also investigated the effect of the reaction time on the coating process. The capillaries were coated with 0.56 M acrylamide and $5.42\times10^{-4}$ M initiator. The EOF was essentially unchanged when the polymerization time increased from 3 h ($6.1\times10^{-6}$ $cm^2 \cdot V^{-1} \cdot s^{-1}$, N = 2, RSD = 7%) to 7 h ($4.7\times10^{-6}$ $cm^2 \cdot V^{-1} \cdot s^{-1}$, N = 2, RSD = 13%). 738 protein groups and 4,086 peptides were identified from 50 ng *E. coli* digest using the coated capillary prepared with 3 h reaction. In contrast, 816 protein groups and 4,084 peptides were identified from 50 ng *E. coli* digest using the coated capillary prepared with 7 h reaction. The identified protein groups and peptides information are listed in Examples.

The results obtained with SCARAFT coated capillaries for bottom-up proteomics of 50 ng *E. coli* digests with an Orbitrap Velos mass spectrometer are much better than our previous report using a commercial LPA coated capillary with 100 ng sample loading amount and the same mass spectrometer.

**Comparison with UPLC-ESI-MS/MS method**

CZE-ESI-MS/MS with the SCARFT coated capillary was compared with UPLC-ESI-MS/MS using an LTQ Orbitrap Velos mass spectrometer with *E. coli* digests. The *E. coli* digests were loaded onto a reverse phase C18 analytical column followed by a 90 min gradient UPLC-ESI-MS/MS analysis, **Figure 6.** Venn diagrams for peptides and protein groups identified by the UPLC and CZE methods are shown in **Figure 2.** 880 protein groups and 4,727 unique peptides were identified with 50 ng *E. coli* digests by using UPLC-ESI-MS/MS method. As mentioned above, 816 protein groups and 4084 peptides were identified from 50 ng *E. coli* digest by using CZE-ESI-MS/MS with the SCARFT coated capillary under similar experimental conditions. The identified protein groups and peptides information are listed in the Examples. 76% of the protein groups and 52% of the peptides identified in the UPLC method were also identified by the CZE method. However, 18% of the protein groups and 40% of peptides that were identified by the CZE method were not identified by UPLC method. The results demonstrate that CZE and UPLC provided complementary peptide level identifications.

**Reproducibility**

The SCARAFT method was used to prepare two LPA coated capillaries. The mean and standard deviation of the electroosmotic mobility of these capillaries were $2.8 \pm 0.3 \times 10^{-6}$ $cm^2 \cdot V^{-1} \cdot s^{-1}$ (N=2), which is nearly an order of magnitude lower than commercial LPA coated capillaries ($2.5\times10^{-5}$ $cm^2 \cdot V^{-1} \cdot s^{-1}$). The detector traces for $\mu_{EOF}$ determination were shown in **Figure 7.**

Reproducibility in proteomics analysis was evaluated by using two of these capillaries with a Q Exactive HF mass spectrometer for single-shot bottom-up proteomics. 990 protein groups and 5,514 peptides were identified from 50 ng *E. coli* digest with the first capillary and 964 protein groups and 5,703 peptides were identified with the second capillary. These numbers of identifications are significantly better than the results reported using single-shot CZE analysis with a commercial LPA-coated capillary and an Orbitrap Fusion mass spectrometer (956 protein groups and 4,741 peptides) from -250 ng *E. coli* digest. The identified protein groups and peptides are listed in the Examples.

**Long term stability testing**

The *Xenopus laevis* digest was injected by pressure every 100 min for 201.7 h (8.4 days) of continuous separation to generate 121 consecutive separations of the *Xenopus laevis* digest. Representative base-peak electropherograms are shown in **Figure 8.** Selected ion electropherograms were generated at six arbitrarily chosen m/z values (m/z = 542.3, 566.1, 577.6, 652.6, 703.6, and 722.2) with mean migration times ranging from 27 to 89 min. The selected ion electropherograms were fit with Gaussian functions using a nonlinear least-squares routine to estimate migration time and peak widths. The relative standard deviation for migration time ranged from 2.0 to 3.0%. This variation is likely dominated by temperature fluctuations in the room; the viscosity of water, and hence electrophoretic and electroosmotic mobilities,

decreases by -2.5% per °C. Peak widths increased roughly linearly with migration time (r = 0.91, n = 744), and the median theoretical plate counts ranged from 240,000 to 600,000 for selected ion electropherograms generated at the six m/z values.

To check the carryover during the separation, a blank analysis was carried out after 121th separation of the *Xenopus laevis* digest by injecting 25 nL of 1 M HAc. The base peak electropherogram is shown in the **Figure 8.** Negligible carryover was observed.

**Analysis of HeLa digest**

We used the SCARAFT coated capillary and a Q Exactive HF mass spectrometer for analysis of a *HeLa* digest. 2,158 protein groups and 10,005 peptides were identified with 25 ng of the HeLa digests. The identified protein groups and peptides are listed in the Examples. A representative electropherogram is shown in **Figure 3.** These numbers of identifications are similar to a previous report from this group using an Orbitrap Fusion mass spectrometer, but the sample amount we now use is 16 times lower. The pI distribution of the identified peptides is shown in **Figure 4.**

The improved performance of the SCARAFT coated capillary was due to the very low EOF, which resulted in a doubling of the separation window compared to the use of a commercially coated LPA capillary. In addition, we believe that the SCARAFT-coated capillaries are more homogeneous and result in less sample loss, which facilitates analysis of small sample loadings. We present the cumulative migration time distribution of the HeLa peptides in **Figure 9.** Though the analysis time was 200 min, most of the peptides (>96%) migrated within 120 min. In addition, the migration time of the peptides *versus* pI is shown in **Figure 10.** Most of the peptides that migrated after 100 min have pI<6, which is expected because peptides with low pI carry low charge and migrate slowly.

Finally, we investigated phosphorylation of the HeLa peptides using phosphoRS 3.0 in Proteome Discoverer software version 1.4. 153 phosphorylation sites (with phosphoRS site probabilities higher than 95%) and 163 phosphopeptides were identified. The modest numbers of phosphorylation sites and phosphopeptides were due to their low abundance, and the results could undoubtedly be improved after enrichment by using titanium (IV) ion affinity chromatography or other forms of enrichment before analysis. The migration time distribution of the phosphopeptides is shown in **Figure 11.** As expected, more than 96% of the identified phosphopeptides migrated after 60 min due to their negatively charged phosphorylation sites. The pI distribution the phosphopeptides is shown in **Figure 12,** most of them (>80%) have a pI lower than 7.

**Preparation of positively charged capillaries by SCARAFT method**

A coated capillary with positively charge was prepared using the SCARAFT method. The anodic EOF was $5.63\times10^{-4}$ $cm^2\cdot V^{-1}\cdot s^{-1}$ (N=3, RSD=0.76%). The reversed direction and the strong EOF confirmed the successful coating process. A mixture of four proteins was separated on the cationic capillary, **Figure 5(A).**

The high EOF produced a short separation window. As a result, only 390 protein groups and 1604 peptides were identified from analysis of 50 ng *E. coli* digest. The identified protein groups and peptides are discussed further in the Examples.

**Preparation of the block copolymer coating**

To prove the living characteristic of the coating prepared by using SCARAFT method, a block polymer coating was prepared. After the first coating (LPA coating) process, the cathodic EOF was $2.42\times10^{-6}$ $cm^2\cdot V^{-1}\cdot s^{-1}$. However, the EOF was reversed to anodic EOF of $5.46\times10^{-4}$ $cm^2\cdot V^{-1}\cdot s^{-1}$ after the second coating (positively charged coating) process. The reversed direction of EOF confirmed the successful preparation of the block copolymer coatings and the living characteristic of the coating prepared by using SCARAFT method. The separation of the four proteins mixture is shown in **Figure 5(B).**

The RDSs of the migration times of myoglobin, ribonuclease A, and lysozyme are listed in **Table 1.** The reproducibility of the migration time produced by the block copolymer coated capillary is more than two-times better than produced by direct positive coating.

355 protein groups and 1,483 peptides were identified from 50 ng *E. coli* digest by using the block polymer coating capillary, which are similar to the direct positively charged coating. The identified protein groups and peptides information are discussed further in the Examples.

**Table 1.** Migration times of intact proteins on two coated capillaries.

| protein | Migration time on capillary with direct positively charged coating/ min | | | | Migration time on the capillary with block copolymer coating / min | | | |
|---|---|---|---|---|---|---|---|---|
| | 1st run | 2nd run | 3rd run | RSD/ % | 1st run | 2nd run | 3rd run | RSD/ % |
| Myoglobin | 12.95 | 13.7 | 13.2 | 2.9 | 11.05 | 10.87 | 10.8 | 1.2 |
| Ribonuclease A | 14.07 | 14.93 | 14.36 | 3.0 | 11.97 | 11.74 | 11.7 | 1.2 |
| Lysozyme | 17.09 | 18.32 | 17.56 | 3.5 | 14.25 | 13.94 | 13.85 | 1.5 |

**Conclusions**

A versatile method based on surface-confined aqueous reversible addition-fragmentation chain transfer (SCARAFT) polymerization reaction for coating capillary was developed. The polymerization reaction mainly takes place on the inner surface of the capillary under the optimized conditions instead of in solution, which avoid the possible clogging and greatly improve the homogeneous of the coating. The EOF of the coated capillary is roughly an order of magnitude lower than that of commercialize LPA coated capillary. The very low EOF results in a long separation window, which produces improved performance for bottom-up proteomics analysis. Various types of coatings could be prepared by simply replace the functional vinyl monomers in the polymerization mixture. By taking advantage of the living characteristic of the coating prepared by the SCARAFT polymerization reaction, capillary coatings based on block co-polymers are easy to prepare. These block co-polymers have great potential to improve the CZE separation performance and expand its range of applications.

The following Examples are intended to illustrate the above invention and should not be construed as to narrow its scope. One skilled in the art will readily recognize that the Examples suggest many other ways in which the invention could be practiced. It should be understood that numerous variations and modifications may be made while remaining within the scope of the invention.

**EXAMPLES**

**Example 1.** Low Electroosmotic Flow Polymer Coatings

**Reagents and Chemicals.** Formic acid (FA), acetic acid (HAc), acrylamide, [2-(methacryloyloxy)ethyl]trimethylammonium chloride solution (80 wt.% in $H_2O$), 4,4 - azobis(4-cyanovaleric acid), cyanomethyl [3-(trimethoxysilyl)propyl] trithiocarbonate, ammonium acetate, bovine pancreas TPCK-treated trypsin, benzyl alcohol, dithiothreitol (DTT), and iodoacetamide (IAA) were purchased from Sigma-Aldrich (St. Louis, USA). Pierce™ HeLa Protein Digest Standard was purchased from Thermo Fisher Scientific (Hanover Park, USA). Methanol was purchased from Honeywell Burdick & Jackson (Wicklow, Ireland). Uncoated fused silica capillary (50 μm i.d.×150 μm o.d.) was purchased from Polymicro Technologies (Phoenix, AZ). Water was deionized by a Nano Pure system from Thermo Scientific (Marietta, OH).

**Preparation of positively charged capillaries by SCARAFT method.** A coated capillary with positively charge was prepared using the SCARAFT method by replacing acrylamide with 0.56 M [2-(methacryloyloxy)ethyl]trimethylammonium chloride.

**Preparation of the block copolymer coating.** First, the LPA coating was prepared by using SCARAFT method as described above. Then, a positively charged coating was prepared on the LPA coating using the SCARAFT method by replacing acrylamide with 0.56 M [2-(methacryloyloxy)ethyl]trimethylammonium chloride.

**CZE-ESI-MS/MS analysis.** The CZE system consisted of two high-voltage power supplies (Spellman CZE 1000R) and an electrokinetically pumped nanospray interface, which coupled the CZE separation capillary to a mass spectrometer. The electrospray emitter was made from a borosilicate glass capillary (1.0 mm o.d. × 0.75 mm i.d., 10 cm long) pulled with a Sutter instrument P-1000 flaming/brown micropipette puller; the size of the emitter opening was 15-20 μm. The electrospray sheath electrolyte was 10% (v/v) methanol with 0.5%FA. The background electrolyte was 1 M HAc in water. The sample was injected by nitrogen pressure. Separation voltage was applied at the injection end of the capillary. 1.6 kV was applied to the sheath flow reservoir for electrospray. Voltage programming was controlled by LabView software. The mass spectrometer's operating parameters are described below.

**Determination of EOF.** The method used for the determination of EOF was similar to a method reported by Williams and Vign (Anal. Chem. 1996, 68, 1174) with minor modification.

**Preparation of *E. coli* sample.** The method used for the preparation of the *E. coli* sample is described below.

**Preparation of *Xenopus laevis* sample.** The method used for the preparation of the *Xenopus laevis* sample

is described below.

**Long term stability testing.** The long term stability of the LPA coated capillary with 50 μm i.d. × 150 μm o.d. × 100 cm was evaluated by using 2 mg/mL of the *Xenopus laevis* proteins digest dissolved in 30 mM $NH_4HCO_3$. A PrinCE Next 840 Series auto sampler (from PrinCE Technologies) coupled to a LTQ XL mass spectrometer (Thermo Scientific) was used to generate 121 consecutive separations of the *Xenopus laevis* digest followed by a blank injection. In this analysis, the 25 nL sample was injected by pressure every 100 min for 201.7 h (8.4 days) of continuous separation. No rinse or regeneration step was performed between injections.

**UPLC-ESI-MS/MS analysis.** A nanoACQUITY UltraPerformance LCH (UPLCH) system (Waters, Milford, MA, USA) with a UPLC BEH 130 C18 column (Waters, 100 μm × 100 mm, 1.7 μm) was coupled to an LTQ Orbitrap Velos mass spectrometer (Thermo Fisher Scientific) for peptide separation and identification. Buffer A (0.1%FA in water) and buffer B (0.1% FA in ACN) were used as mobile phases for gradient separation. Peptides were automatically loaded onto a commercial C18 reversed phase column (Waters, 100 μm × 100 mm, 1.7 μm particle, BEH130C18, column temperature 40°C) and flushed with 2% buffer B for 10 min at a flow rate of 1 μL/min, then followed by gradient: 10-11 min, 2-8% B; 11-71 min, 8-30% B; 71-72 min, 30-80% B; 72-77 min, 80% B; 77-78 min, 80-2% B; 78-90 min, 2% B. The eluted peptides from the C18 column were pumped through a capillary tip for electrospray. The MS parameters were the same as that used for CZE-ESI-MS/MS analysis.

**Mass Spectrometer Operating Parameters.** An LTQ-Orbitrap Velos mass spectrometer (Thermo Fisher Scientific) was used to optimize the coating conditions and for the comparison between CZE and UPLC methods. The electrospray voltage was 1.6 kV, and the ion transfer tube temperature was held at 300 °C. The S-Lens RF level was 50.00. The mass spectrometer was programmed in data-dependent mode. Full MS scans were acquired in the Orbitrap mass analyzer over the m/z 380-1800 range with resolution of 30,000 (m/z 400) and the number of microscans was set to 1. The target value was 1.00E+06, and maximum injection time was 500 ms. For MS/MS scans, the 20 most intense peaks with charge state ≥ 2 were sequentially isolated and further fragmented in the collision-induced dissociation mode following one full MS scan. The normalized collision energy was 35%.

A Q Exactive HF mass spectrometer (Thermo Scientific) was used to evaluate the reproducibility of the coating method and for analysis of the HeLa digest. The mass spectrometer was programmed in data-dependent mode. A top 20 method was used. The S-lens RF level was set at 60, and heated capillary at 300 °C. Full scan resolution was set to 60,000 at m/z 200. Full scan target was 3.00E+06 with a maximum fill time of 50 ms. Mass range was set to m/z 350-1,800. Target value for fragment scans was set at 1.00E+05, and intensity threshold was kept at 1.00E+05. Isolation width was set at 1.4 Th. A fixed first mass of 100 was used. Normalized collision energy was set at 28. The proteins mixture was analyzed in an intact mode. Full MS scans were acquired in the Orbitrap over the m/z 600-2000. The three most intense peaks with charge state ≥5 were selected in data dependent fashion for fragmentation. The AGC target value for MS1 was 1.00E+06 with a maximum injection time of 100 ms, whereas the AGC target value for MS2 scans is 5.00E+05 and a maximum injection time is 200 ms. Seven and three microscans were used in MS1 and MS2 scans, respectively. An exclusion window of ± 10 ppm was constructed around the monoisotopic peak of each selected precursor for 5 s. Other parameters were the same as above.

A LTQ XL mass spectrometer (Thermo Scientific) was used to evaluate the long term stability of the LPA coated capillary. Full MS scans were acquired over the 380-1800 m/z range.

**Database Searching.** Database searching of the raw files was performed in Proteome Discoverer 1.4 with MASCOT 2.5. The SwissProt databases with taxonomy as *E. coli* and *Homo sapiens* were used as database for *E. coli* cell lysate and HeLa cell line proteome digest, respectively. Database searching for the reversed database was also performed to evaluate the false discovery rate. The database searching parameters included full tryptic digestion and allowed up to two missed cleavages, the precursor mass tolerance was set at 10 ppm, and fragment mass tolerance was 0.5 Da for the data obtained on the LTQ-Orbitrap Velos mass spectrometer and 0.05 Da for the data obtained on the Q Exactive HF instrument. Carbamidomethylation (C) was set as a fixed modification. Oxidation (M) and deamidated (NQ) were set as variable modifications. On the peptide level, peptide confidence value as high was used to filter the peptide identification, and the corresponding false discovery rate on peptide level was less than 1%. On the protein level, protein grouping was enabled.

**Example 2.** Sample Preparation

**Preparation of *E. coli* sample.** Solid lysogeny broth (LB) was used to make the agar plates for the E. coli culture. Solid LB was prepared by dissolving 3 g of NaCl, 3 g of tryptone, 1.5 g of yeast extract, and 6 g of agar in 300 mL of deionized water. Liquid LB medium (without agar) was also prepared for E. coli culture by mixing 10 g of NaCl, 10 g of tryptone, and 5 g of yeast extract in 1 L of deionized water. All media, plates, and other utensils and flasks were autoclaved before use. Frozen cultures of E. coli (Dh5- Alpha) were thawed and plated on the prepared agar plates. After incubation at 37 °C for 24 h, single colonies were isolated and grown in tubes with 4 mL of liquid LB medium and incubated in a shaker at 37 °C overnight. When the tubes' contents turned opaque, the liquid medium was transferred

into new flasks and shaken overnight at 37 °C. The liquid LB medium with E. coli was centrifuged, and the resulting E. coli pellets were washed with phosphate-buffered saline three times. Then, the E. coli pellets were suspended in 8 M urea and 100 mM Tris-HCl (pH 8.0) buffer supplemented with protease inhibitor and sonicated for 15 min on ice for cell lysis. The lysate was centrifuged at 18 000 g for 15 min, and the supernatant was collected. The protein concentration was measured by the BCA method. An aliquot of protein (900 μg) was precipitated by cold acetone overnight at -20 °C. After centrifugation, the pellet was washed again with cold acetone. The resulting protein pellet was dried at room temperature.

The dried E. coli proteins (120 μg) were dissolved in 100 μL of 100 mM NH4HCO3 (pH 8) with 8 M urea and denatured at 37 °C for 1 h. After addition of 10 μL of 1 M DTT, the mixture was incubated at 37 °C for 1 h to reduce disulfide bonds. Subsequently, 20 μL of 2 M IAA was added to the mixture, which was incubated in the dark at room temperature for 30 min. Then, 900 μL of 100 mM NH4HCO3 (pH 8) was added to reduce the concentration of urea below 1 M. Finally, the treated proteins were digested by incubation with trypsin at a trypsin/protein ratio of 1/25 (w/w) for 16 h at 37 °C. The digests were acidified with 10% TFA (v/v) to terminate the reaction. The tryptic digests were desalted with C18 SepPak columns (Waters, Milford, MA), followed by lyophilisation with a vacuum concentrator (Thermo Fisher Scientific, Marietta, OH). The dried protein digests were dissolved in 50 mM FA.

**Preparation of *Xenopus laevis* sample.** All animal procedures were performed according to protocols approved by the University of Notre Dame Institutional Animal Care and Use Committee. *Xenopus laevis* eggs at 32-cell stage (a total of 20 eggs) were collected into an Eppendorf tube. After the egg culture buffer was removed, 500 μL of mammalian Cell-PE LB buffer plus complete protease inhibitors was immediately added into the Eppendorf tube, followed by vortexing for 1 min to preliminarily lyse the eggs. Then, the eggs were homogenized by a PowerGen Model 125 homogenizer (Fisher Scientific) for 2 min on ice and further sonicated for 20 min (5 min ×4) on ice with a Branson Sonifier 250 (VWR Scientific, Batavia, IL) to lyse the eggs completely. Then, the lysate was centrifuged at 18 000g for 20 min, and three layers were obtained in the Eppendorf tube: top layer (lipid), medium layer (protein), and pellet. Finally, the protein layer was transferred to another Eppendorf tube, and a small fraction of the protein solution was subjected to protein concentration measurement with the bicinchoninic acid method. Aliquoted 200 μg protein and purified by using acetone precipitation. 6 times sample volume of cold acetone was added into the protein solution, and the mixture was stored at -20 °C overnight. After centrifugation at 18 000 g for 20 min, the supernatant was removed and the protein pellet was washed with cold acetone again to completely remove the detergent in the lysis buffer. After centrifugation again, the supernatant was removed and the protein pellet was kept in a fume hood at room temperature for ~5 min to dry the sample. The sample was stored at -80 °C before use.

**Determination of EOF.** The method used for the determination of EOF is similar as previously reported with minor modification (Anal. Chem. 1996, 68, 1174). First, the capillary is filled with the separation buffer (1 M HAc). Next, a plug of a neutral marker, benzyl alcohol prepared with 1 M HAc, is injected into the capillary for time $t_{inj}$, (band N1). Then, a plug of separation buffer is loaded into the capillary under pressure for time $t_{tr}$. Second, another band of the neutral marker solution is injected (band N2) again for time $t_{inj}$. Then, the capillary is switched to the background electrolyte, and the band N2 is transferred in the capillary under the injection pressure for time $t_{tr}$. Third, the separation voltage, $V_{separation}$, is applied for time $t_{migr}$. During this time, bands N1 and N2 move toward the cathode (for a cathodic EO flow) with mobilities equal to $\mu_{EOF}$. Then, after Vseparation returns to zero, a third plug of neutral marker is injected into the capillary (band N3) for time $t_{inj}$. Finally, the injection pressure is applied again onto the background electrolyte vial and data acquisition is initiated simultaneously to record the passage of all three bands. Then, $\mu_{EOF}$ can be calculated as:

$$\frac{[(t_{N3}-t_{N1})-(t_{N2}-t_{N1})]\cdot L^2}{V_{separation}\cdot t_{migr}\cdot(t_{N3}+\frac{t_{inj}}{2})} \quad (1)$$

Where $t_{N1}$, $t_{N2}$ and $t_{N3}$ are the observed mobilization times for band N1, N2, and N3. L is the length of the separation capillary.

While specific embodiments have been described above with reference to the disclosed embodiments and examples, such embodiments are only illustrative and do not limit the scope of the invention. Changes and modifications can be made in accordance with ordinary skill in the art without departing from the invention in its broader aspects as defined in the following claims.

The invention has been described with reference to various specific and preferred embodiments and techniques. However, it should be understood that many variations and modifications may be made while remaining within the scope of the invention, which is defined in the claims.

## Claims

1. A polymer coated separation capillary comprising:

   a) a fused silica capillary;
   b) a coating comprising a chain transfer moiety covalently bonded to the inner surface of the fused silica capillary; and
   c) a substituted polyethylene polymer covalently bonded to the chain transfer moiety
   wherein the polyethylene units of the polymer are substituted by -C(=O)G, wherein G is $NR_2$, or OR; R is H, $(C_1-C_6)$alkyl, or $(C_1-C_6)$alkyl-$N(R^a)_3X$; each $R^a$ is independently H, $(C_1-C_6)$alkyl, or aryl; and is X is a counter ion; wherein the polymer coated separation capillary has an electroosmotic flow of about $0.1 \times 10^{-6}$ $cm^2$ $V^{-1}$ $s^{-1}$ to about $10 \times 10^{-4}$ $cm^2$ $V^{-1}$ $s^{-1}$ for capillary zone electrophoresis, and the coating is uncontaminated by metals and free radical scavengers.

2. The separation capillary of claim 1 wherein the electroosmotic flow is about $0.1 \times 10^{-6}$ $cm^2$ $V^{-1}$ $s^{-1}$ to about $10 \times 10^{-6}$ $cm^2$ $V^{-1}$ $s^{-1}$.

3. The separation capillary of claim 1 wherein -C(=O)G is $-C(=O)NH_2$; or $-C(=O)OCH_2N(CH_3)_3X$.

4. The separation capillary of claim 1 wherein the polymer comprises a random polymer or block copolymer of $-C(=O)NH_2$ substituted ethylene units and $-C(=O)OCH_2N(CH_3)_3$ substituted ethylene units.

5. The separation capillary of claim 1 wherein the separation capillary comprises about 200,000 to about 800,000 theoretical plates.

6. The separation capillary of claim 1 wherein the chain transfer moiety comprises $--SiJ_2(CH_2)_3SC(=S)S-$, wherein each J is independently H, G, or halo.

7. The separation capillary of claim 1 wherein the separation capillary comprises about 200,000 to about 800,000 theoretical plates, the electroosmotic flow is about $1 \times 10^{-6}$ $cm^2$ $V^{-1}$ $s^{-1}$ to about $10 \times 10^{-6}$ $cm^2$ $V^{-1}$ $s^{-1}$, and -C(=O)G is $-C(=O)NR_2$;
   wherein, optionally, R is H.

8. The separation capillary of claim 1 wherein the coating comprises a polymer of Formula I or Formula II:

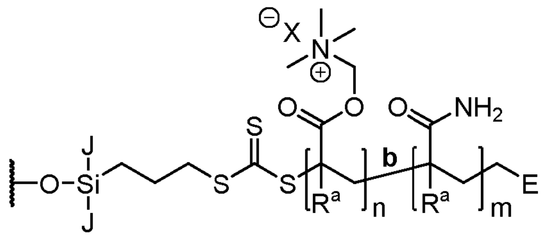

(I),

or

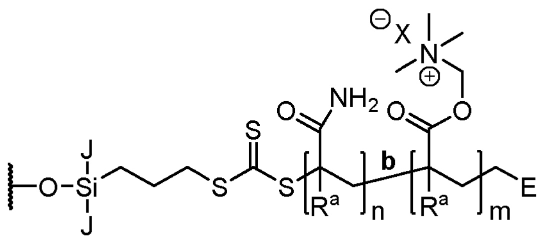

(II);

wherein

J is methoxy, ethoxy or halo;
X is halo;
E is H, aryl, alkyl, cyano;
n is 0 to 10,000; and
m is 0 to 10,000, wherein n and m cannot both be 0.

**9.** A method of fabricating the polymer coated separation capillary of claim 1 comprising:

a) contacting the inner surface of the fused silica capillary with a chain transfer reagent to provide a covalently modified inner surface of the fused silica capillary;
b) contacting the modified inner surface with an aqueous mixture of a radical initiator, a substituted vinyl monomer, and an optional second monomer; and
c) initiating a living radical polymerization by heating or irradiating the mixture; thereby fabricating the polymer coated separation capillary.

**10.** The method of claim 9 wherein the aqueous mixture comprises a buffer.

**11.** The method of claim 9 wherein the concentration of the radical initiator is about $10^{-5}$ molar to about $10^{-3}$ molar; or wherein the concentration of the substituted vinyl monomer is about 0.1 molar to about 2 molar.

**12.** The method of claim 9 wherein the polymer coated separation capillary has a neutral charge; or wherein the polymer coated separation capillary is positively charged.

**13.** The method of claim 9 wherein the polymer coated separation capillary is coated on its inner surface with a block copolymer.

**14.** The method of claim 9 wherein the substituted vinyl monomer is substituted by - C(=O)G, and the optional second monomer is substituted by -C(=O)G;
wherein, optionally, a living radical polymerization in the polymer coated separation capillary is reinitiated by repeating steps b) and c) with the second monomer.

**15.** The method of claim 9 wherein the polymer coated separation capillary comprises an inner surface coated with a polymer of Formula IA or Formula IIA:

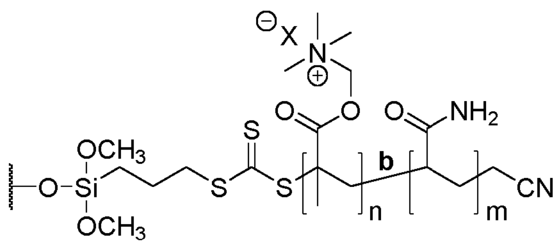
(IA),

or

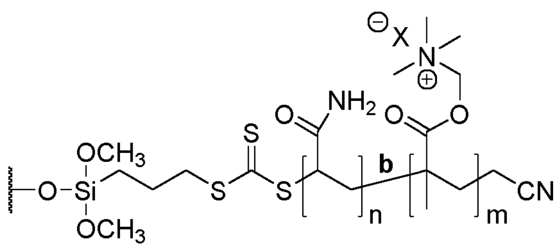
(IIA);

wherein

X is halo;
n is 0 to 10,000; and
m is 0 to 10,000 wherein n and m cannot both be 0;
wherein, optionally, the polymer coated separation capillary performs reproducible separations by capillary zone electrophoresis of at least 5,000 identifiable peptides for at least 100 hours of continuous operation.

**Patentansprüche**

1. Polymerbeschichtete Trennkapillare, die Folgendes umfasst:

   a) eine Quarzglaskapillare;
   b) eine Beschichtung, die eine Kettentransfereinheit umfasst, die kovalent zu der inneren Oberfläche der Quarzglaskapillare gebunden ist; und
   c) ein substituiertes Polyethylenpolymer, das kovalent zu der Kettentransfereinheit gebunden ist, wobei die Polyethyleneinheiten des Polymers durch -C(=O)G substituiert sind, wobei G $NR_2$ oder OR ist; R H, $(C_1\text{-}C_6)$-Alkyl oder $(C_1\text{-}C_6)$-Alkyl-$N(R^a)_3X$ ist;
   jedes $R^a$ unabhängig H, $(C_1\text{-}C_6)$-Alkyl oder Aryl ist; und X ein Gegenion ist;
   wobei die polymerbeschichtete Trennkapillare einen elektroosmotischen Fluss von etwa $0{,}1 \times 10^{-6}$ $cm^2$ $V^{-1}$ $s^{-1}$ bis etwa $10 \times 10^{-4}$ $cm^2$ $V^{-1}$ $s^{-1}$ für Kapillarzonenelektrophorese hat und die Beschichtung nicht durch Metalle und Radikalfänger kontaminiert ist.

2. Trennkapillare nach Anspruch 1, wobei der elektroosmotische Fluss etwa $0{,}1 \times 10^{-6}$ $cm^2$ $V^{-1}$ $s^{-1}$ bis etwa $10 \times 10^{-6}$ $cm^2$ $V^{-1}$ $s^{-1}$ beträgt.

3. Trennkapillare nach Anspruch 1, wobei -C(=O)G $-C(=O)NR_2$ oder $-C(=O)OCH_2N(CH_3)_3X$ ist.

4. Trennkapillare nach Anspruch 1, wobei das Polymer ein statistisches Polymer oder Block-Copolymer von $-C(=O)NR_2$-substituierten Ethyleneinheiten und $-C(=O)OCH_2N(CH_3)_3$-substituierten Ethyleneinheiten umfasst.

5. Trennkapillare nach Anspruch 1, wobei die Trennkapillare etwa 200 000 bis etwa 800 000 theoretische Böden umfasst.

6. Trennkapillare nach Anspruch 1, wobei die Kettentransfereinheit $--SiJ_2(CH_2)_3SC(=S)S-$ umfasst, wobei jedes J unabhängig H, G oder Halogen ist.

7. Trennkapillare nach Anspruch 1, wobei die Trennkapillare etwa 200 000 bis etwa 800 000 theoretische Böden umfasst, der elektroosmotische Fluss etwa $1 \times 10^{-6}$ $cm^2$ $V^{-1}$ $s^{-1}$ bis etwa $10 \times 10^{-6}$ $cm^2$ $V^{-1}$ $s^{-1}$ beträgt und -C(=O)G $-C(=O)NR_2$ ist;
   wobei optional R H ist.

8. Trennkapillare nach Anspruch 1, wobei die Beschichtung ein Polymer der Formel I oder Formel II umfasst:

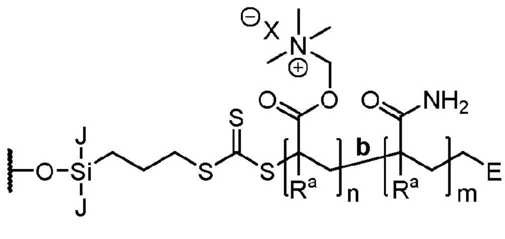

(I)

oder

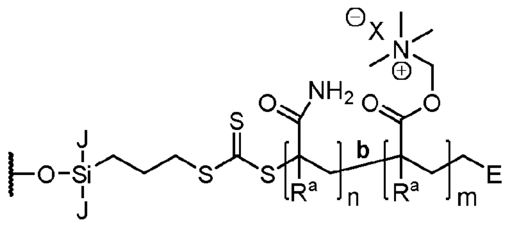

(II);

wobei

J Methoxy, Ethoxy oder Halogen ist;
X Halogen ist;
E H, Aryl, Alkyl, Cyano ist;
n 0 bis 10 000 ist; und
m 0 bis 10 000 ist, wobei n und m nicht beide 0 sein können.

**9.** Verfahren zur Herstellung der polymerbeschichteten Trennkapillare nach Anspruch 1, das Folgendes umfasst:

a) Inkontaktbringen der inneren Oberfläche der Quarzglaskapillare mit einem Kettentransferreagenz, um eine kovalent modifizierte innere Oberfläche der Quarzglaskapillare bereitzustellen;
b) Inkontaktbringen der modifizierten inneren Oberfläche mit einer wässrigen Mischung eines Radikalstarters, eines substituierten Vinylmonomers und eines optionalen zweiten Monomers; und
c) Starten einer lebenden radikalischen Polymerisation durch Erhitzen oder Bestrahlen der Mischung;

wodurch die polymerbeschichtete Trennkapillare hergestellt wird.

**10.** Verfahren nach Anspruch 9, wobei die wässrige Mischung einen Puffer umfasst.

**11.** Verfahren nach Anspruch 9, wobei die Konzentration des Radikalstarters etwa $10^{-5}$ molar bis etwa $10^{-3}$ molar ist; oder wobei die Konzentration des substituierten Vinylmonomers etwa 0,1 molar bis etwa 2 molar ist.

**12.** Verfahren nach Anspruch 9, wobei die polymerbeschichtete Trennkapillare eine neutrale Ladung aufweist; oder wobei die polymerbeschichtete Trennkapillare positiv geladen ist.

**13.** Verfahren nach Anspruch 9, wobei die polymerbeschichtete Trennkapillare auf ihrer inneren Oberfläche mit einem Block-Copolymer beschichtet ist.

**14.** Verfahren nach Anspruch 9, wobei das substituierte Vinylmonomer durch -C(=O)G substituiert ist und das optionale zweite Monomer durch -C(=O)G substituiert ist;
wobei optional eine lebende radikalische Polymerisation in der polymerbeschichteten Trennkapillare erneut durch Wiederholen der Schritte b) und c) mit dem zweiten Monomer gestartet wird.

**15.** Verfahren nach Anspruch 9, wobei die polymerbeschichtete Trennkapillare eine innere Oberfläche umfasst, die mit einem Polymer der Formel IA oder Formel IIA beschichtet ist:

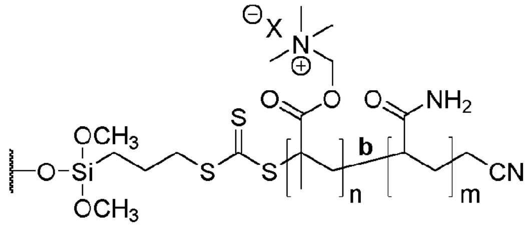

(IA)

oder

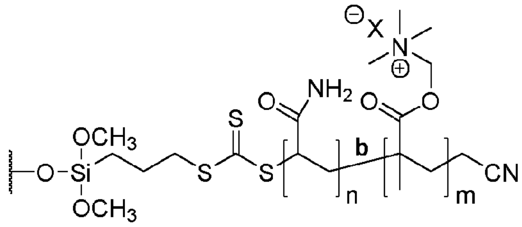

(IIA);

wobei

X Halogen ist;
n 0 bis 10 000 ist; und
m 0 bis 10 000 ist, wobei n und m nicht beide 0 sein können;
wobei optional die polymerbeschichtete Trennkapillare reproduzierbare Trennungen durch Kapillarzonenelektrophorese von mindestens 5 000 identifizierbaren Peptiden für mindestens 100 Stunden kontinuierlichen Betrieb ausführt.

## Revendications

**1.** Capillaire de séparation revêtu de polymère, comprenant :

a) un capillaire en silice pyrogénée ;
b) un revêtement comprenant un motif de transfert de chaîne lié de manière covalente à la surface interne du capillaire en silice pyrogénée ; et
c) un polymère de polyéthylène substitué lié de manière covalente au motif de transfert de chaîne, où les motifs de polyéthylène du polymère sont substitués par - C(=O)G, où G est $NR_2$, ou OR ; R est H, ($C_1$-$C_6$)alkyle, ou ($C_1$-$C_6$)alkyl-N($R^a$)$_3$X ;
chaque $R^a$ est indépendamment H, ($C_1$-$C_6$)alkyle, ou aryle ; et X est un contre-ion ;
le capillaire de séparation revêtu de polymère présentant un flux électroosmotique allant d'environ $0,1 \times 10^{-6}$ $cm^2$ $V^{-1}$ $s^{-1}$ à environ $10 \times 10^{-4}$ $cm^2$ $V^{-1}$ $s^{-1}$ pour l'électrophorèse capillaire de zone, et le revêtement n'est pas contaminé par des métaux et des piégeurs de radicaux libres.

**2.** Capillaire de séparation selon la revendication 1, dans lequel the flux électroosmotique va d'environ $0,1 \times 10^{-6}$ $cm^2$ $V^{-1}$ $s^{-1}$ à environ $10 \times 10^{-6}$ $cm^2$ $V^{-1}$ $s^{-1}$.

**3.** Capillaire de séparation selon la revendication 1, dans lequel -C(=O)G est - C(=O)$NH_2$ ; ou -C(=O)$OCH_2N(CH_3)_3X$.

**4.** Capillaire de séparation selon la revendication 1, dans lequel le polymère comprend un polymère aléatoire ou un copolymère bloc de motifs éthylène à substitution -C(=O)$NH_2$ et de motifs éthylène à substitution -C(=O)$OCH_2N(CH_3)_3$.

**5.** Capillaire de séparation selon la revendication 1, le capillaire de séparation comprenant d'environ 200 000 à environ 800 000 plateaux théoriques.

**6.** Capillaire de séparation selon la revendication 1, dans lequel le motif de transfert de chaîne comprend --$SiJ_2(CH_2)_3SC(=S)S$-, où chaque J est indépendamment H, G, ou halogéno.

**7.** Capillaire de séparation selon la revendication 1, le capillaire de séparation comprenant d'environ 200 000 à environ 800 000 plateaux théoriques, le flux électroosmotique allant d'environ $1 \times 10^{-6}$ $cm^2$ $V^{-1}$ $s^{-1}$ à environ $10 \times 10^{-6}$ $cm^2$ $V^{-1}$ $s^{-1}$, et - C(=O)G étant -C(=O)$NR_2$ ;
où, éventuellement, R est H.

**8.** Capillaire de séparation selon la revendication 1, dans lequel le revêtement comprend un polymère de Formule I ou de Formule II :

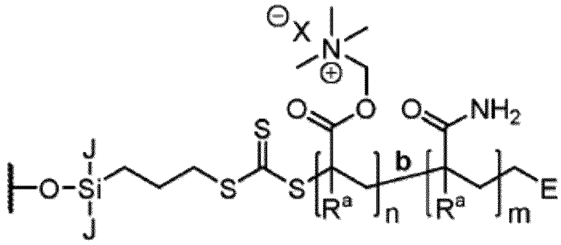

(I),

ou

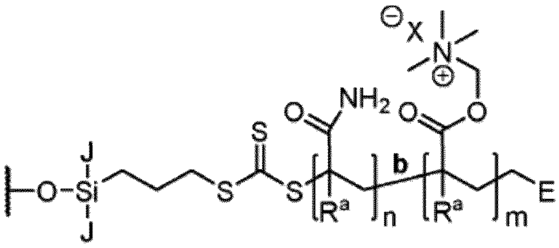

(II);

dans laquelle

J est méthoxy, éthoxy ou halogéno ;
X est halogéno ;
E est H, aryle, alkyle, cyano ;
n va de 0 à 10 000 ; et
m va de 0 à 10 000, où n et m ne peuvent pas tous deux valoir 0.

**9.** Méthode de fabrication du capillaire de séparation revêtu de polymère selon la revendication 1, comprenant :

a) la mise en contact de la surface interne du capillaire en silice pyrogénée avec un réactif de transfert de chaîne afin de fournir une surface interne, modifiée de manière covalente, du capillaire en silice pyrogénée ;
b) la mise en contact de la surface interne modifiée avec un milieu aqueux d'un initiateur radicalaire, d'un monomère de vinyle substitué, et d'un deuxième monomère facultatif ; et
c) l'initiation d'une polymérisation radicalaire vivante par le chauffage ou l'irradiation du mélange ;

pour fabriquer ainsi le capillaire de séparation revêtu de polymère.

**10.** Méthode selon la revendication 9, dans laquelle le milieu aqueux comprend un tampon.

**11.** Méthode selon la revendication 9, dans laquelle la concentration en initiateur radicalaire va d'environ $10^{-5}$ molaire à environ $10^{-3}$ molaire ; ou
où la concentration en monomère de vinyle substitué va d'environ 0,1 molaire à environ 2 molaire.

**12.** Méthode selon la revendication 9, dans laquelle le capillaire de séparation revêtu de polymère présente une charge neutre ; ou
où le capillaire de séparation revêtu de polymère est positivement chargé.

**13.** Méthode selon la revendication 9, dans laquelle le capillaire de séparation revêtu de polymère est revêtu sur sa surface interne par un copolymère bloc.

**14.** Méthode selon la revendication 9, dans laquelle le monomère de vinyle substitué est substitué par -C(=O)G, et le deuxième monomère facultatif est substitué par - C(=O)G ;
où, éventuellement, une polymérisation radicalaire vivante dans le capillaire de séparation revêtu de polymère est réinitiée par la répétition des étapes b) et c) avec le deuxième monomère.

**15.** Méthode selon la revendication 9, dans laquelle le capillaire de séparation revêtu de polymère comprend une surface interne revêtue par un polymère de Formule IA ou de Formule IIA :

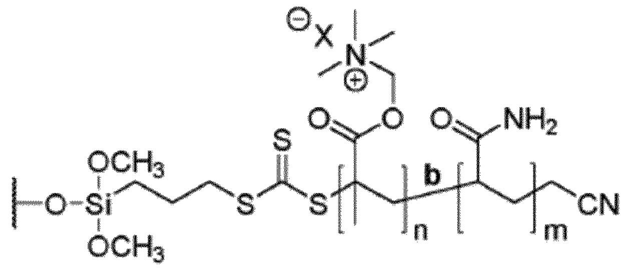

(IA),

ou

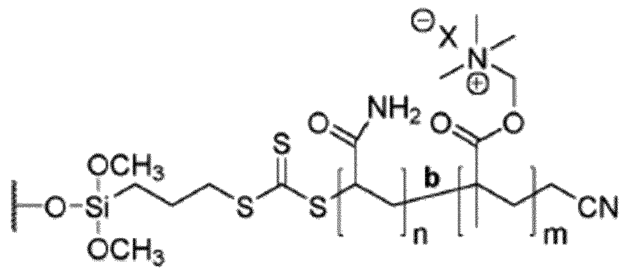

(IIA);

dans laquelle

X est halogéno ;
n va de 0 à 10 000 ; et
m va de 0 à 10 000, où n et m ne peuvent pas tous deux valoir 0 ;

où, éventuellement, le capillaire de séparation revêtu de polymère met en oeuvre des séparations reproductibles par électrophorèse capillaire de zone d'au moins 5000 peptides identifiables pendant au moins 100 heures de fonctionnement continu.

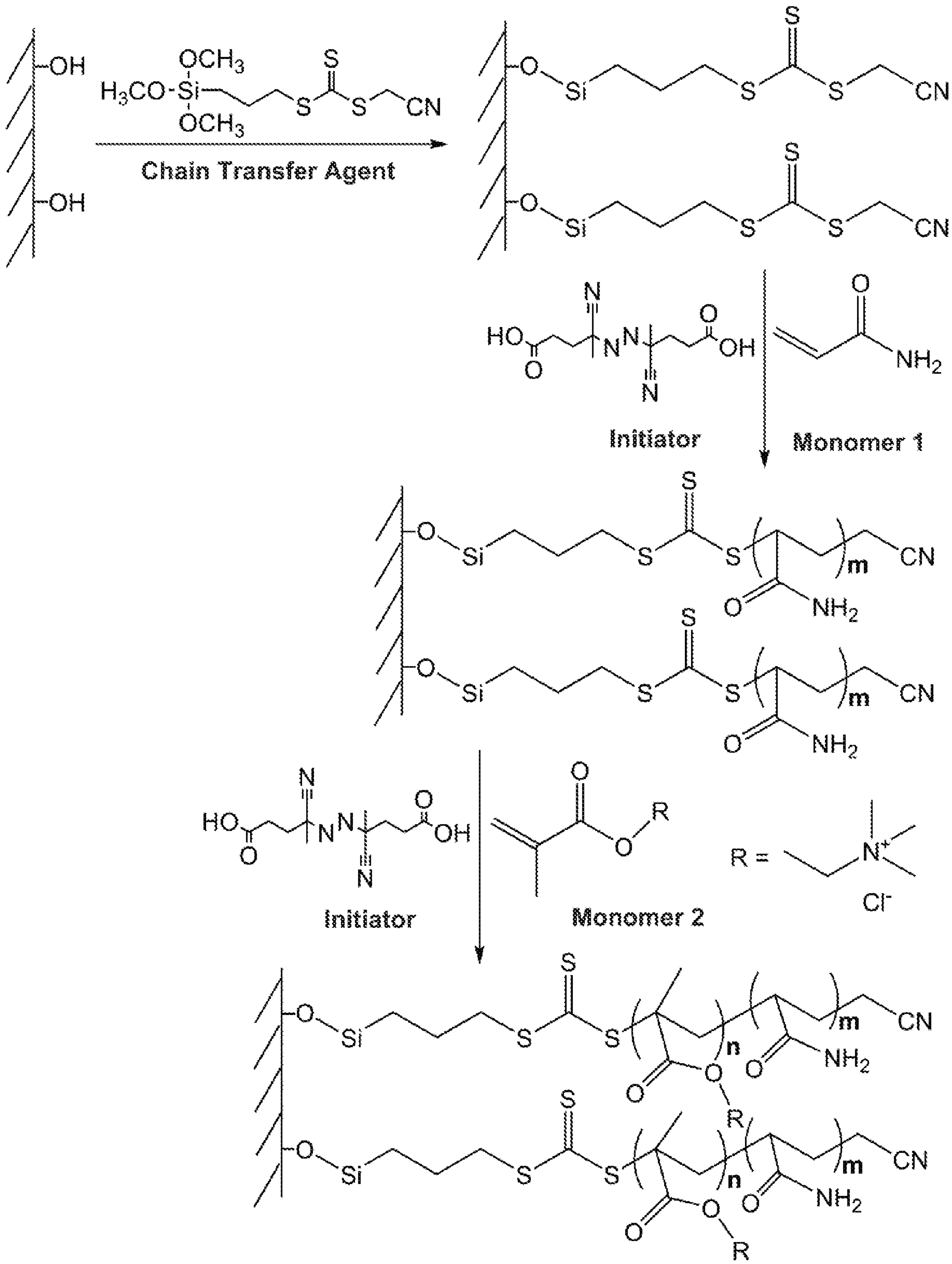
Chain Transfer Agent
Initiator
Monomer 1
Initiator
Monomer 2

*Fig. 1*

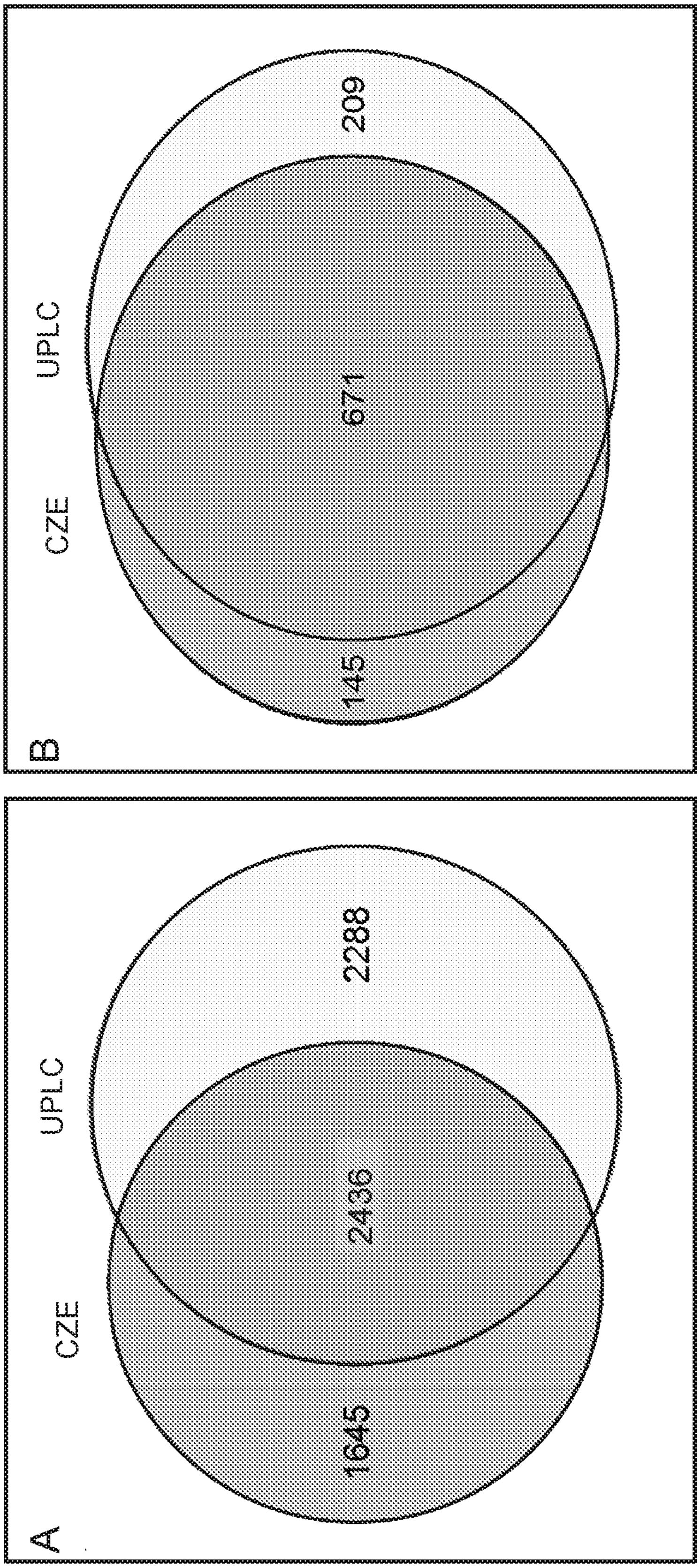


*Fig. 2*

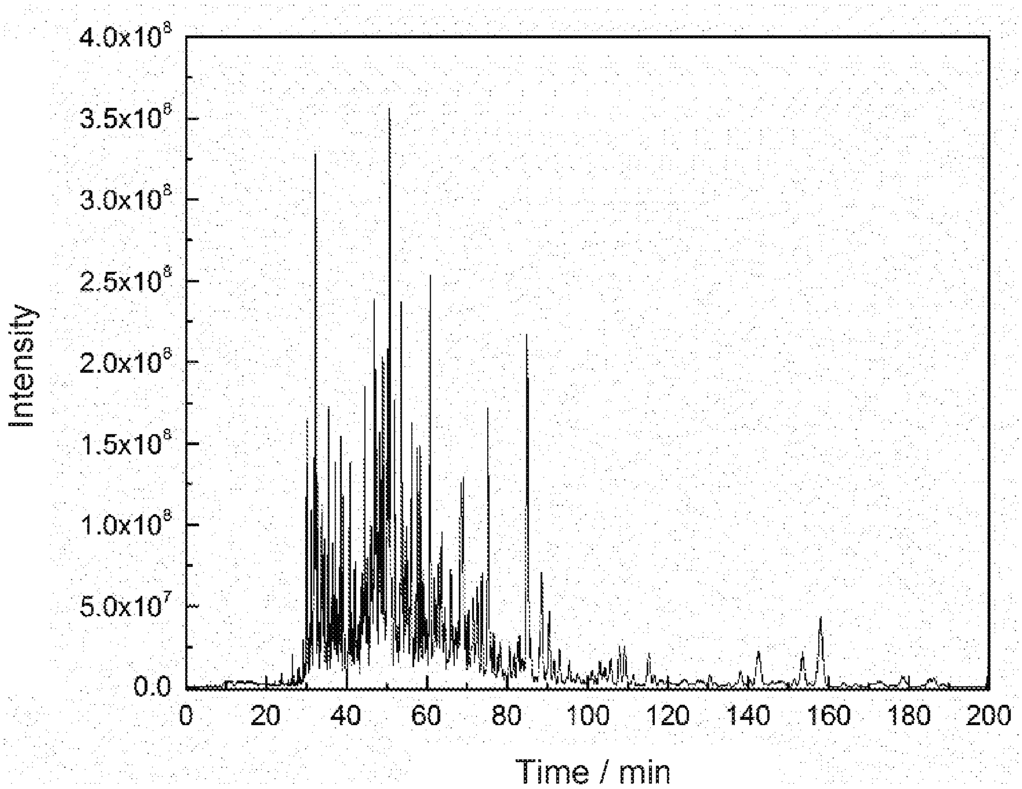
Intensity
4.0x10^8
3.5x10^8
3.0x10^8
2.5x10^8
2.0x10^8
1.5x10^8
1.0x10^8
5.0x10^7
0.0
0 20 40 60 80 100 120 140 160 180 200
Time / min

*Fig. 3*

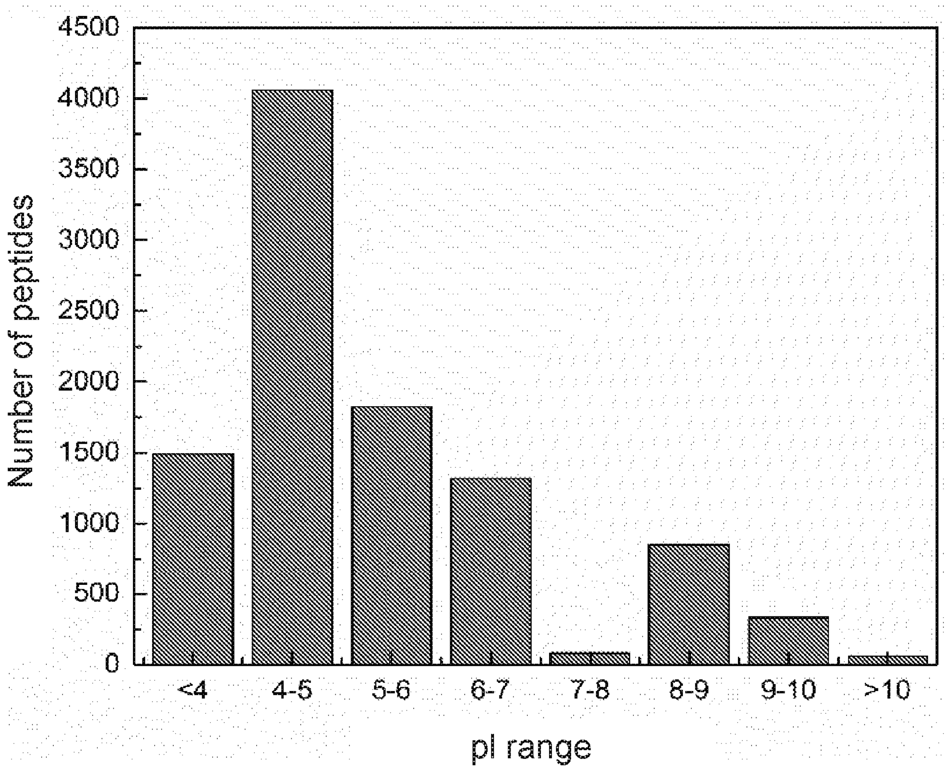
Number of peptides
4500
4000
3500
3000
2500
2000
1500
1000
500
0
<4 4-5 5-6 6-7 7-8 8-9 9-10 >10
pI range

*Fig. 4*

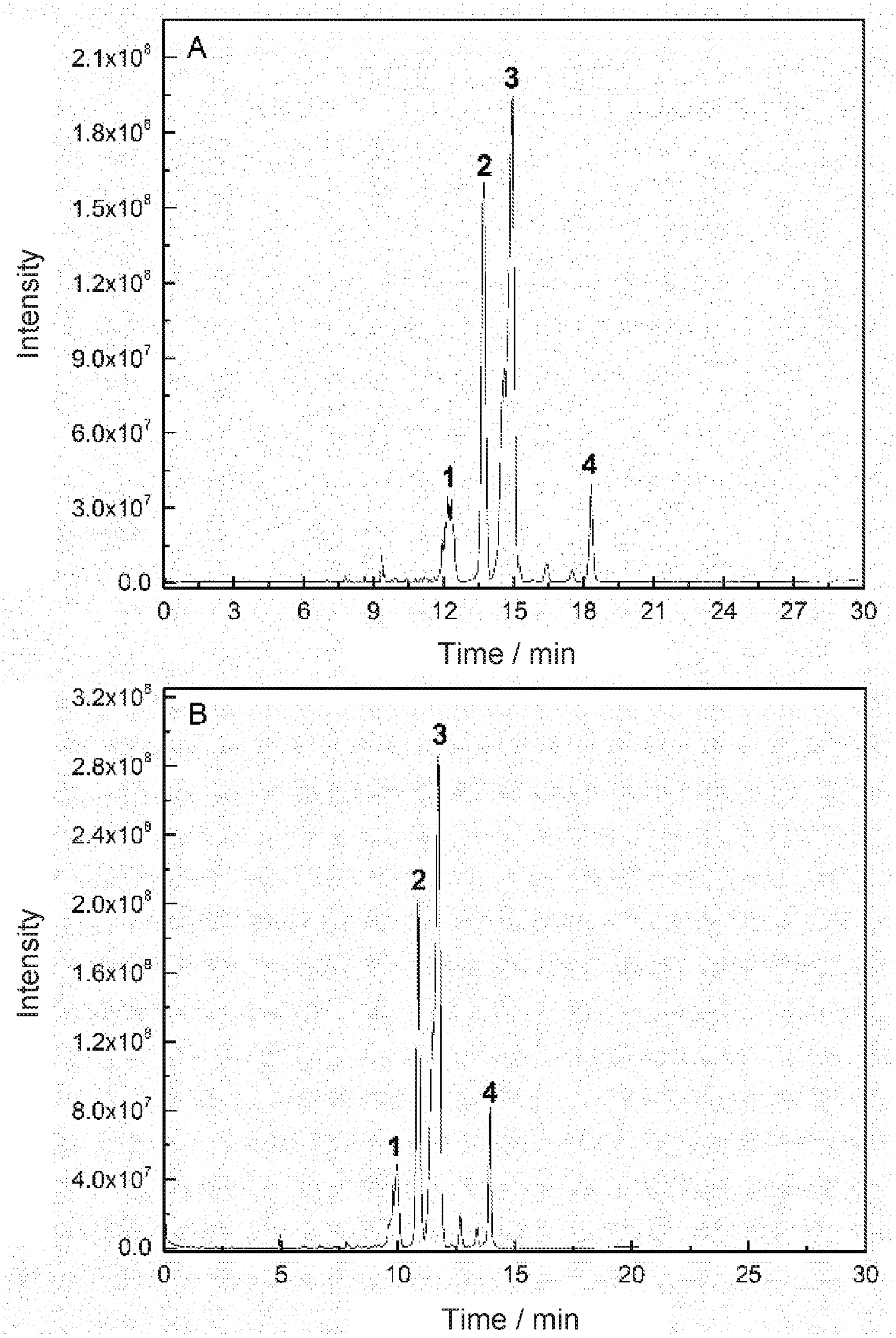


*Fig. 5*

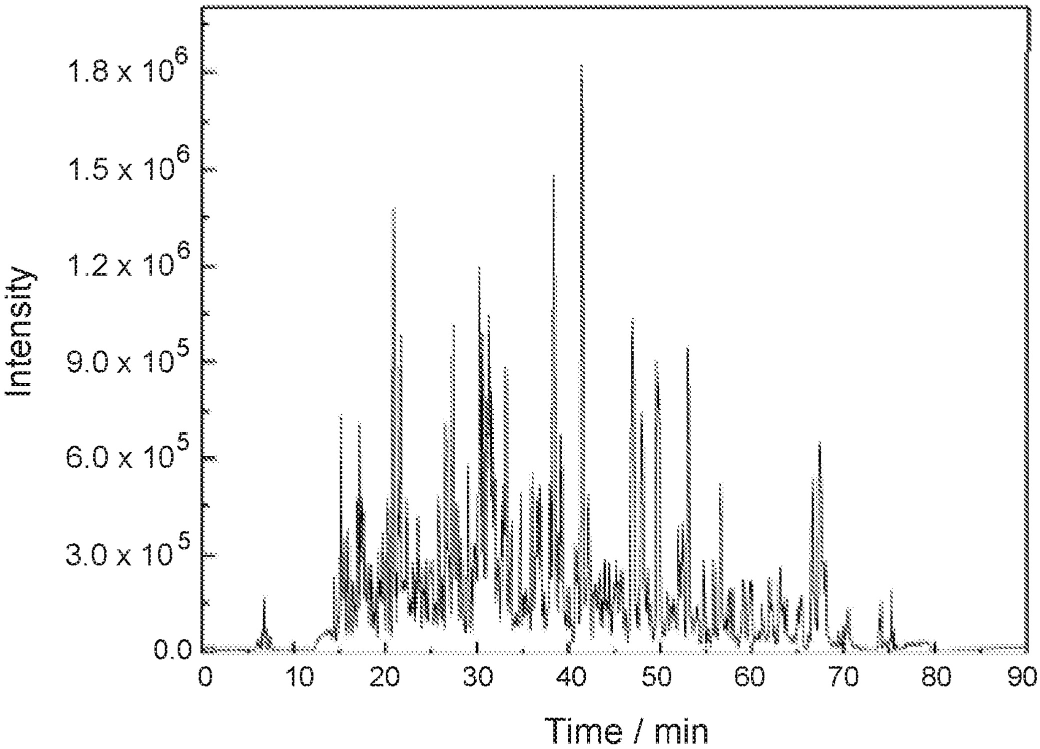

Intensity
1.8 x 10^6
1.5 x 10^6
1.2 x 10^6
9.0 x 10^5
6.0 x 10^5
3.0 x 10^5
0.0
0 10 20 30 40 50 60 70 80 90
Time / min


*Fig. 6*

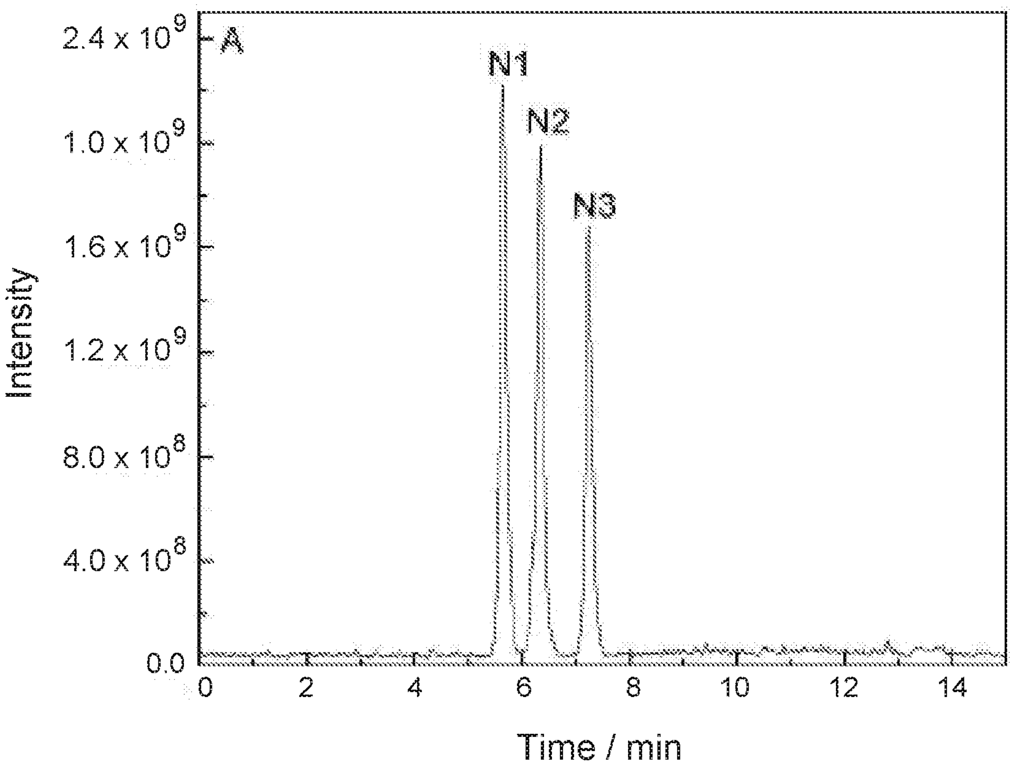

A
N1
N2
N3
Intensity
2.4 x 10^9
1.0 x 10^9
1.6 x 10^9
1.2 x 10^9
8.0 x 10^8
4.0 x 10^8
0.0
0 2 4 6 8 10 12 14
Time / min


*Fig. 7*

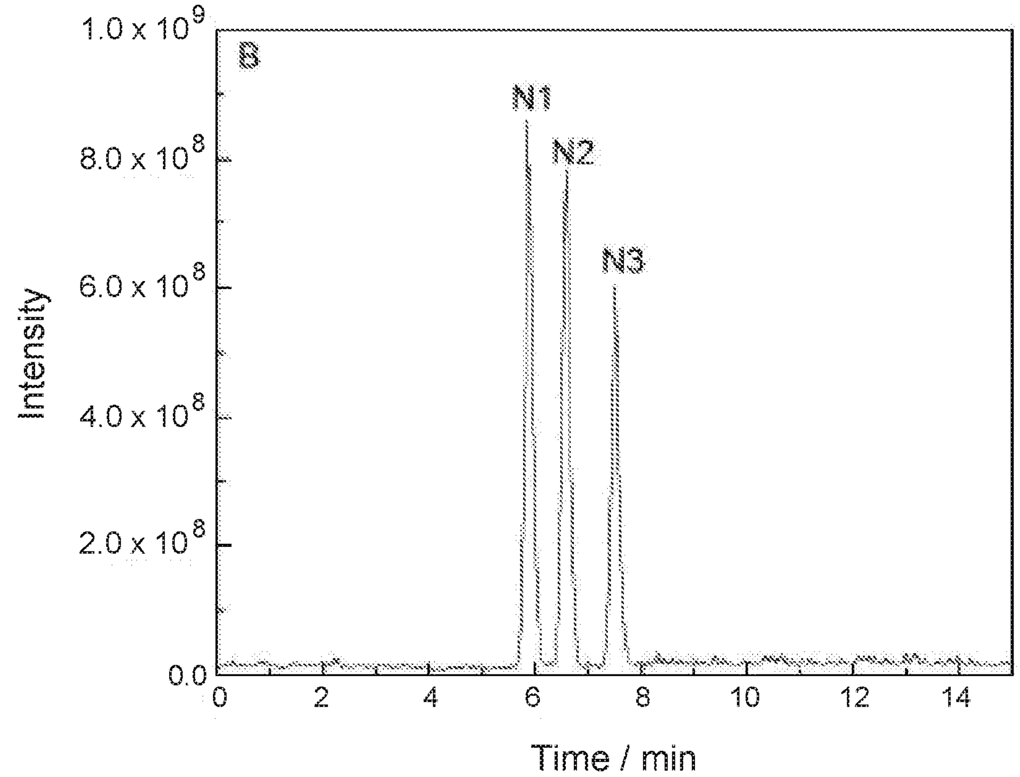
B
N1
N2
N3
Intensity
1.0 x 10^9
8.0 x 10^8
6.0 x 10^8
4.0 x 10^8
2.0 x 10^8
0.0
0
2
4
6
8
10
12
14
Time / min

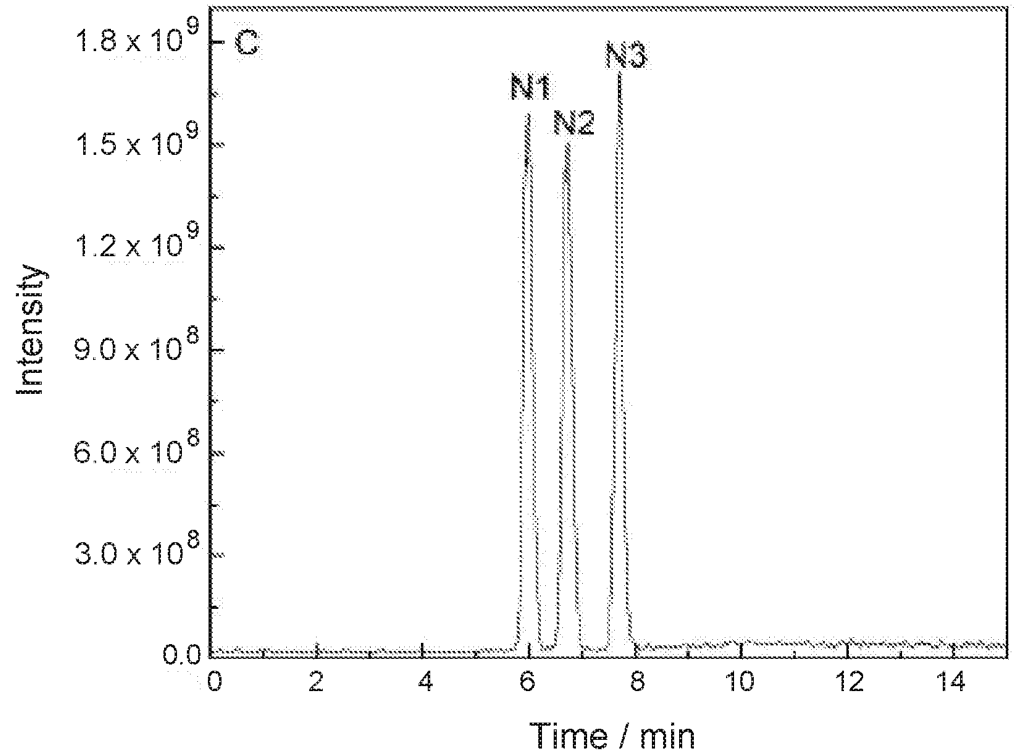
C
N1
N2
N3
Intensity
1.8 x 10^9
1.5 x 10^9
1.2 x 10^9
9.0 x 10^8
6.0 x 10^8
3.0 x 10^8
0.0
0
2
4
6
8
10
12
14
Time / min

*Fig. 7 (cont.)*

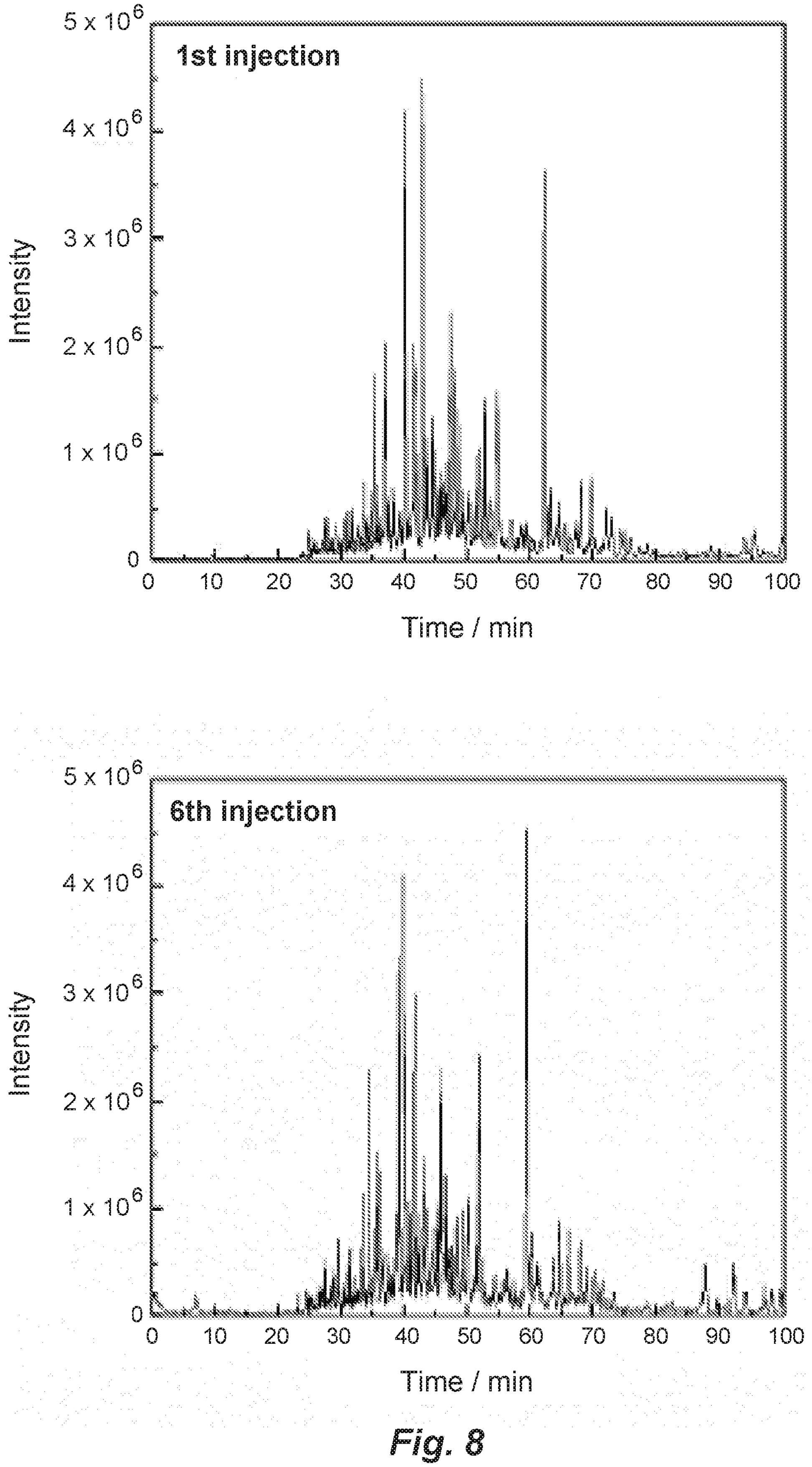
1st injection
Intensity
5 x 10^6
4 x 10^6
3 x 10^6
2 x 10^6
1 x 10^6
0
0 10 20 30 40 50 60 70 80 90 100
Time / min
6th injection
Intensity
5 x 10^6
4 x 10^6
3 x 10^6
2 x 10^6
1 x 10^6
0
0 10 20 30 40 50 60 70 80 90 100
Time / min

*Fig. 8*

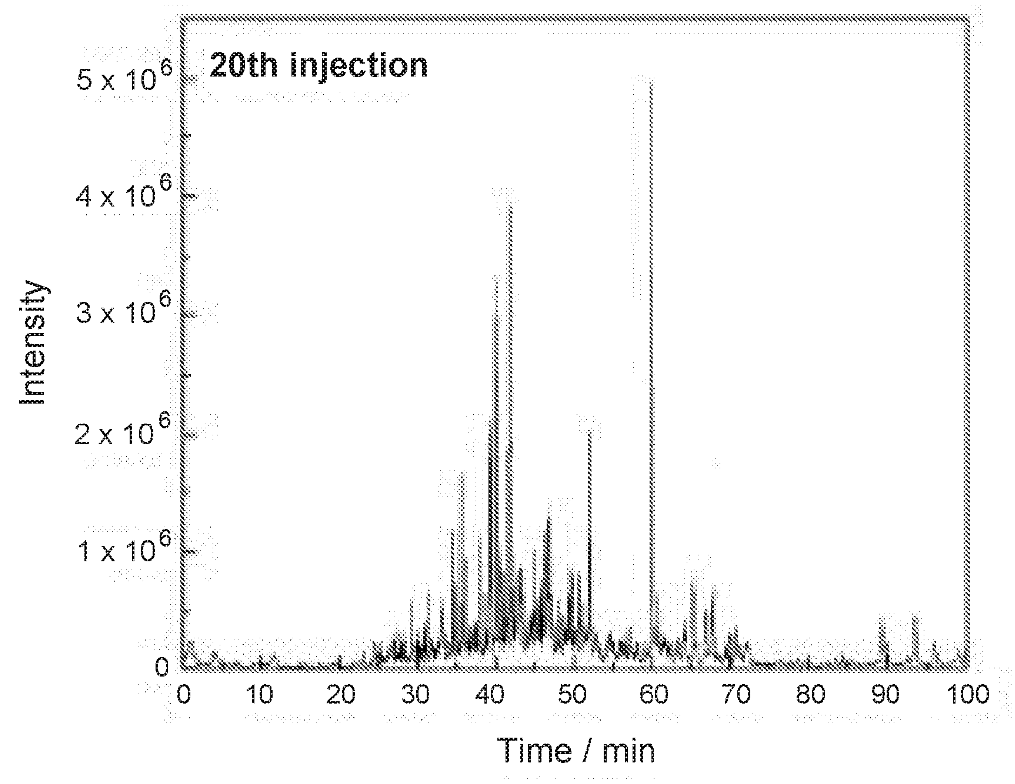
20th injection
Intensity
5 x 10^6
4 x 10^6
3 x 10^6
2 x 10^6
1 x 10^6
0
0 10 20 30 40 50 60 70 80 90 100
Time / min

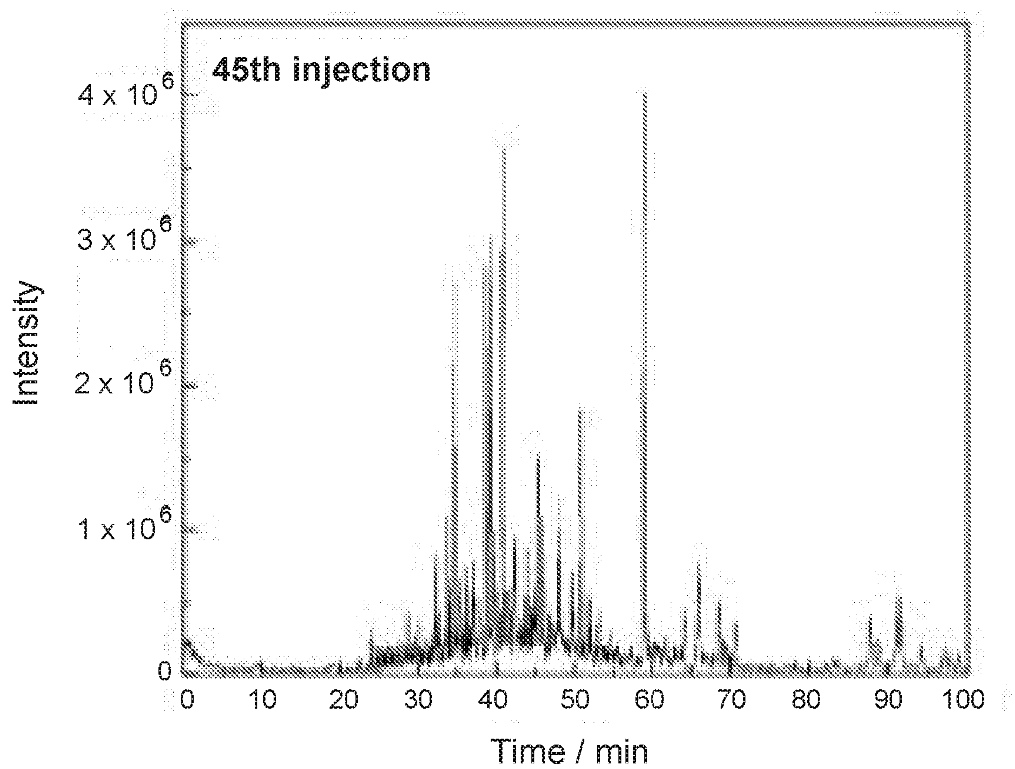
45th injection
Intensity
4 x 10^6
3 x 10^6
2 x 10^6
1 x 10^6
0
0 10 20 30 40 50 60 70 80 90 100
Time / min

*Fig. 8 (cont.)*

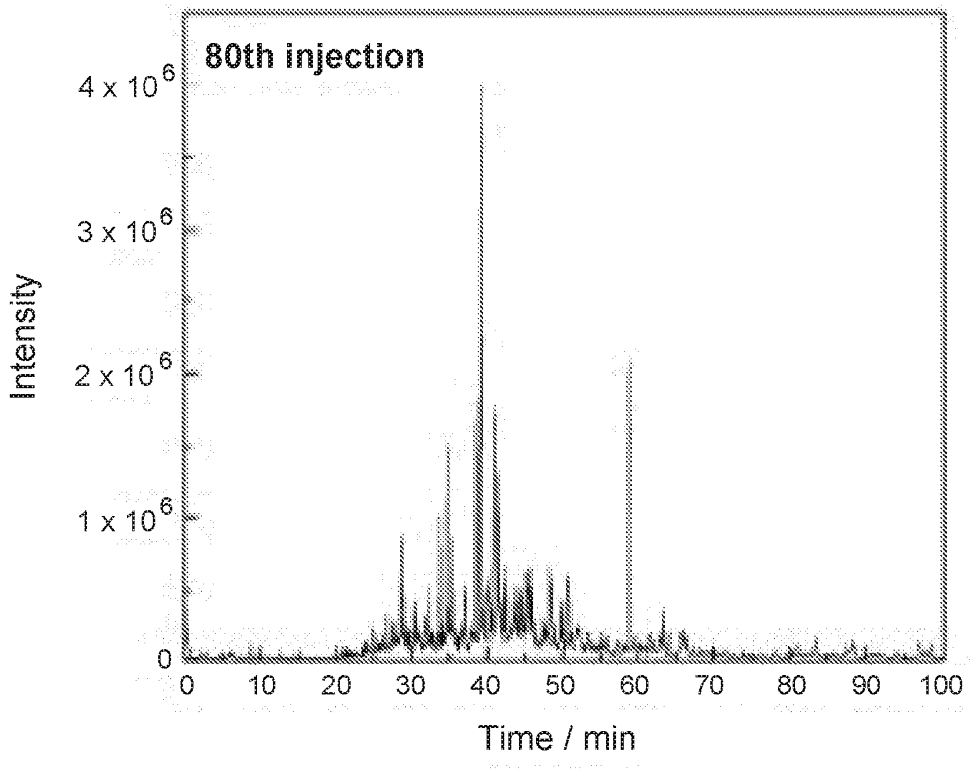
80th injection
4 x 10^6
3 x 10^6
2 x 10^6
1 x 10^6
0
Intensity
0 10 20 30 40 50 60 70 80 90 100
Time / min

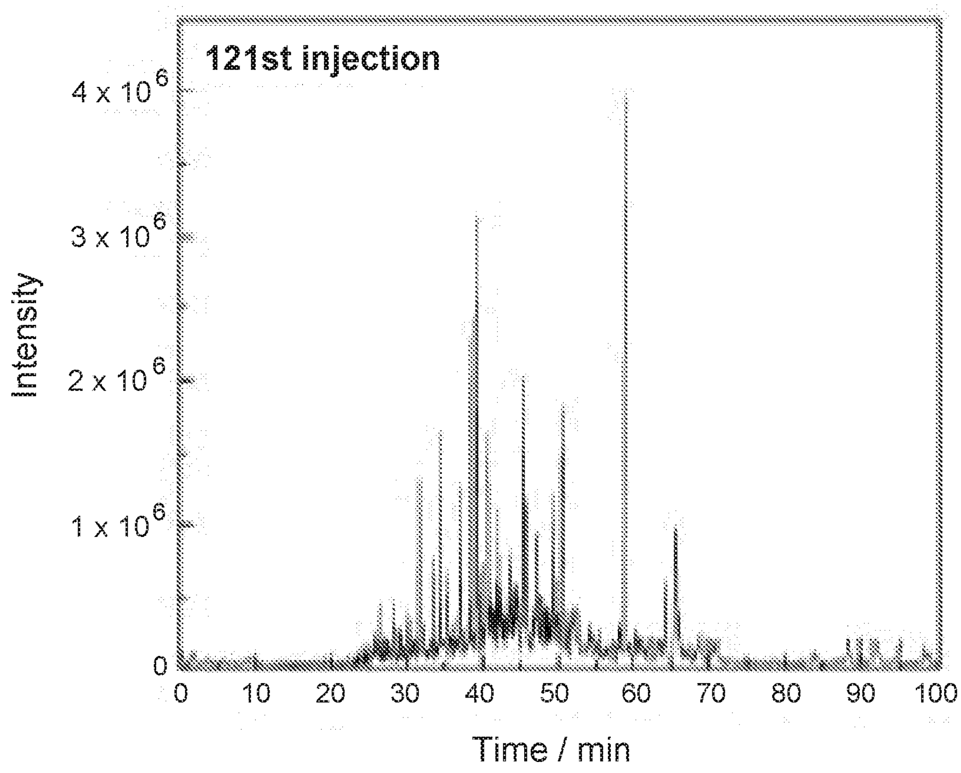
121st injection
4 x 10^6
3 x 10^6
2 x 10^6
1 x 10^6
0
Intensity
0 10 20 30 40 50 60 70 80 90 100
Time / min

*Fig. 8 (cont.)*

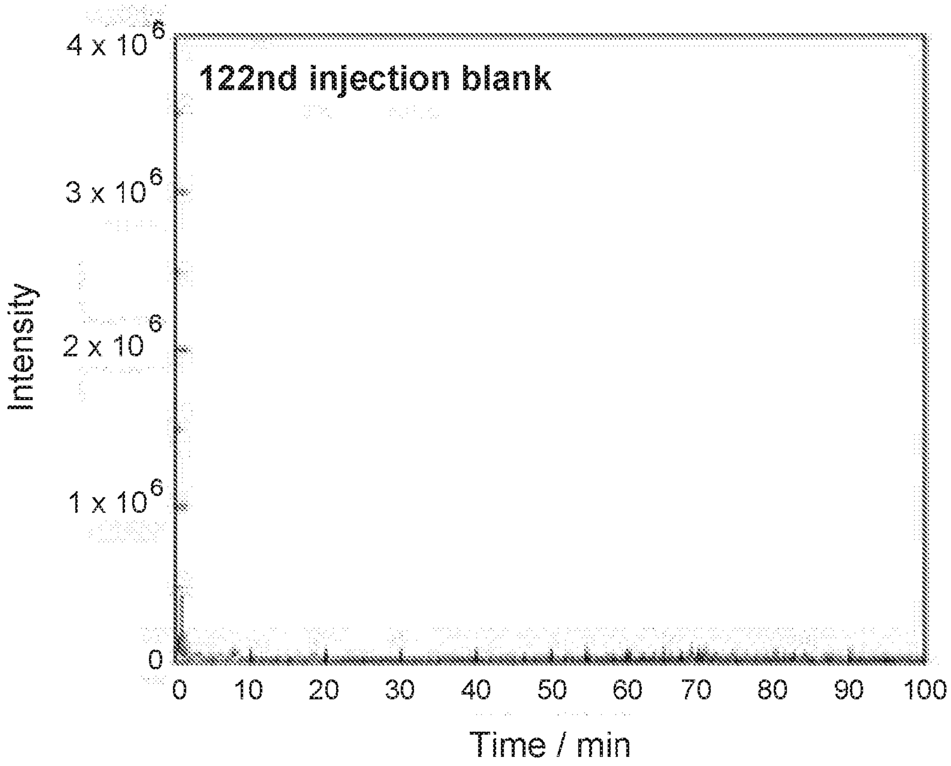


*Fig. 8 (cont.)*

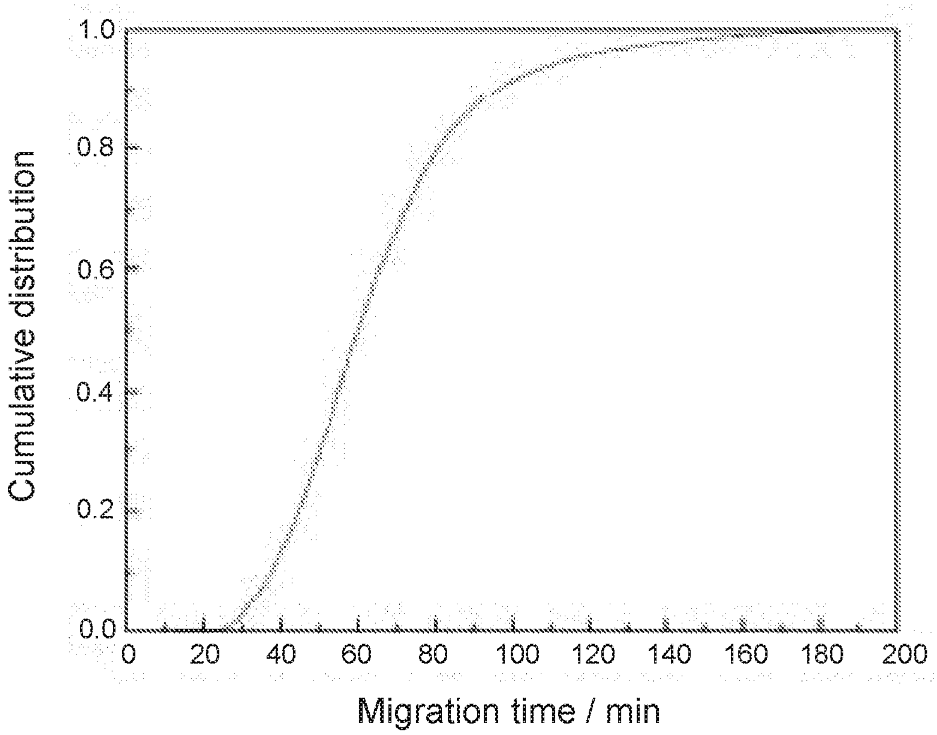


*Fig. 9*

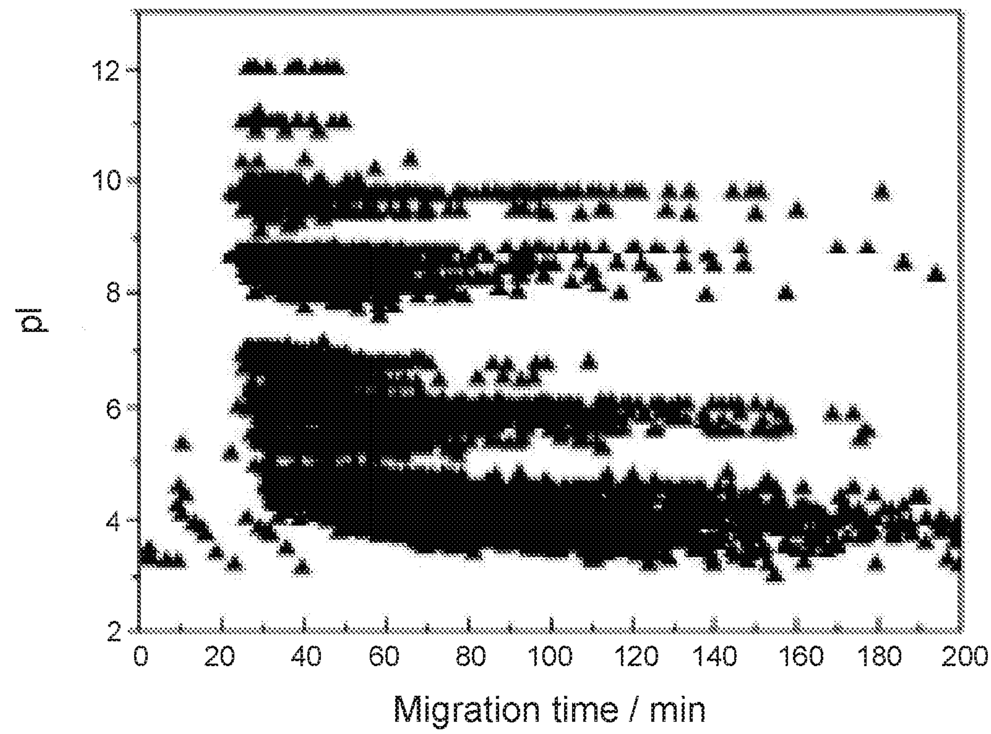
pI
12
10
8
6
4
2
0
20
40
60
80
100
120
140
160
180
200
Migration time / min

*Fig. 10*

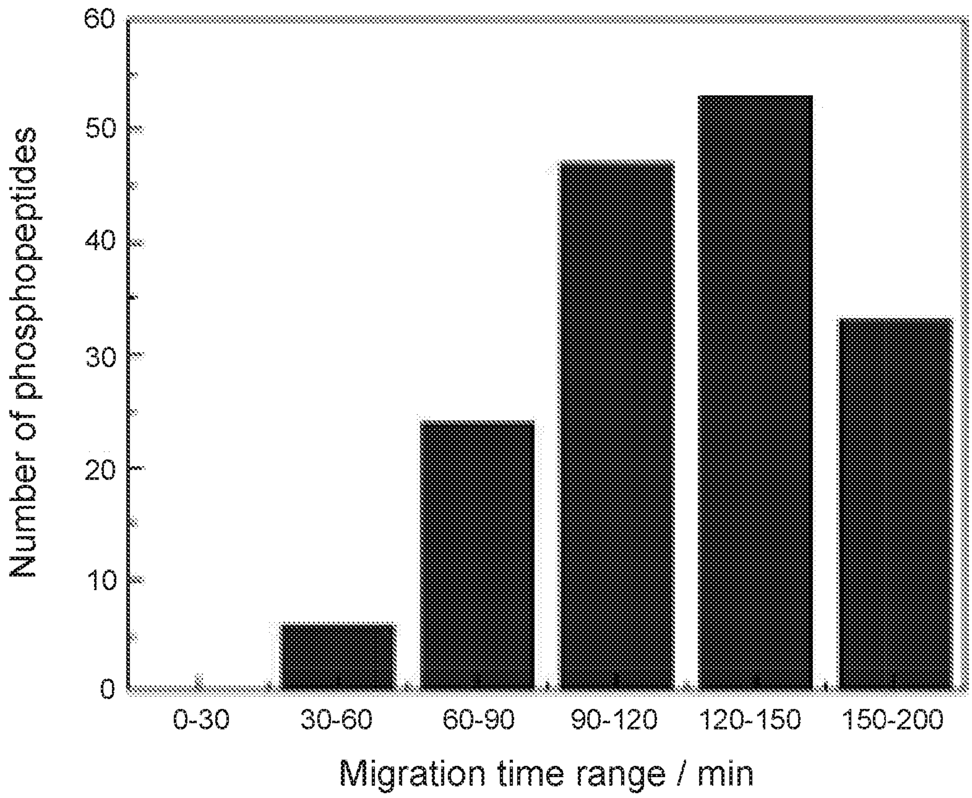
Number of phosphopeptides
60
50
40
30
20
10
0
0-30
30-60
60-90
90-120
120-150
150-200
Migration time range / min

*Fig. 11*

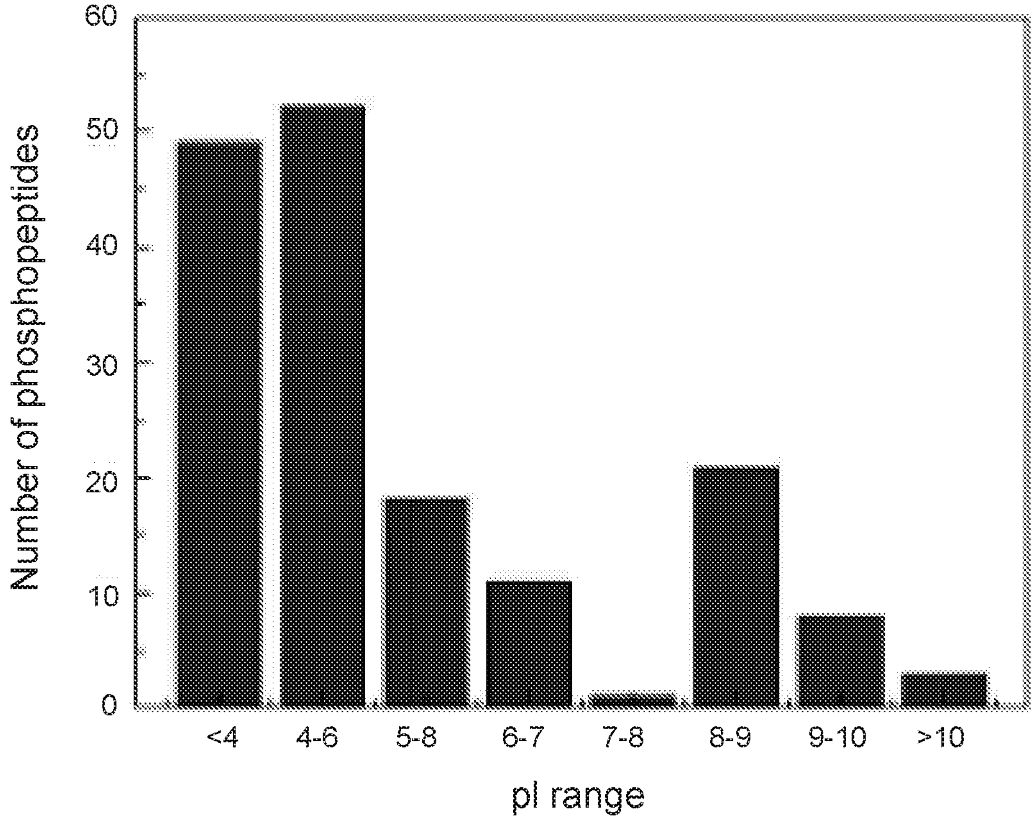
60
50
40
30
20
10
0
Number of phosphopeptides
<4
4-6
5-8
6-7
7-8
8-9
9-10
>10
pI range

*Fig. 12*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62488892
- WO 0109204 A
- US 6074541 A


### Non-patent literature cited in the description


- *Anal. Chem.,* 1998, vol. 70, 4023
- **ALI ; CHEONG.** *J. Sep. Sci.,* 2015, vol. 38, 1763
- *Analytical Chemistry,* vol. 89 (12), 6774-6780
- concerns surface-confined living radical polymerization for coatings in capillary electrophoresis. **XUEYING HUANG et al.** Analytical Chemistry. American Chemical Society, vol. 70, 4023-4029
- **JEAN-FRANCOIS BAUSSARD et al.** concerns new chain transfer agents for reversible addition-fragmentation chain transfer (RAFT) polymerisation in aqueous solution. *Polymer,* vol. 45 (11), 3615-3626
- concerns cross-linked polymer coatings for capillary electrophoresis and application to analysis of basic proteins, acidic proteins, and inorganic ions. **SRINIVASAN K et al.** Analytical Chemistry. American Chemical Society, vol. 69, 2798-2805
- **R.J. LEWIS.** Hawley's Condensed Chemical Dictionary. John Wiley & Sons, 2001
- *Macromol. Rapid Commun.,* 2011, vol. 32, 958
- **WILLIAMS ; VIGN.** *Anal. Chem.,* 1996, vol. 68, 1174
- *Anal. Chem.,* 1996, vol. 68, 1174